# EUROPEAN PATENT SPECIFICATION

(11) **EP 1 028 942 B1**
(45) Date of publication and mention of the grant of the patent: **01.09.2010**
(21) Application number: 98958887.6
(22) Date of filing: 03.11.1998
(51) Int. Cl.: C07C 255/29, A61K 31/275, A61K 31/33

(54) **DIPEPTIDE NITRILES**
DIPEPTIDE NITRILE
NITRILES DE DIPEPTIDES

(30) Priority: 05.11.1997 GB 9723407; 05.12.1997 US 985973
(43) Date of publication of application: 23.08.2000
(73) Proprietor: Novartis AG, 4056 Basel (CH); Novartis Pharma GmbH, 1230 Wien (AT)
(72) Inventor: ALTMANN, Eva, CH-4153 Reinach (CH); BETSCHART, Claudia, Tsukuba Ibaraki Pref. (JP); GOHDA, Keigo, Ushiku, Ibaraki Pref. (JP); HORIUCHI, Miyuki, Tsukuba, Ibaraki Pref. (JP); LATTMANN, Rene, CH-4102 Binningen (CH); MISSBACH, Martin, CH-5073 Gipf-Oberfrick (CH); SAKAKI, Junichi, Takatsu-ku, Kawasaki Kanagawa Pref. (JP); TAKAI, Michihiro, Tsukuba Ibaraki Pref. (JP); TENO, Naoki, Ushiku, Ibaraki Pref. (JP); COWEN, Scott, Douglas, Branchburg, NJ 08876 (US); GREENSPAN, Paul, David, New Providence, NJ 07974 (US); MCQUIRE, Leslie, Wighton, Warren, NJ 07059 (US); TOMMASI, Ruben, Alberto, Whitehouse Station, NJ 08889 (US); VAN DUZER, John, Henry, Asbury, NJ 08802 (US)
(74) Representative: de Weerd, Petrus G.W.
(86) International application number: PCT/EP1998/006937
(87) International publication number: WO 1999/024460

(56) References cited:
- EP-A- 0 611 756
- WO-A-97/27200
- WO-A-98/22433
- BE-A- 662 788
- DUFOUR E ET AL: "Peptide Aldehydes and Nitriles as Transition State Analog Inhibitors of Cysteine Proteases" BIOCHEMISTRY (BICHAW,00062960);1995; VOL.34 (28); PP.9136-43, XP002107811 National Research Council of Canada;Biotechnology Research Institute; Montreal; H4P 2R2; PQ; Can. (CA)
- KHALID, M. ET AL: "N,N'-disubstituted L-isoglutamines as novel cancer chemotherapeutic agents" DRUGS EXP. CLIN. RES. (1987), 13(SUPPL. 1), 57-60 CODEN: DECRDP;ISSN: 0378-6501, 1987, XP002108052
- SUZUE, SEIGO ET AL: "Hepatic agents. I. Synthesis of aminoacyl (and hydroxyacyl)aminoacetonitriles" CHEM. PHARM. BULL. (TOKYO) (1968), 16(8), 1417-32 CODEN: CPBTAL,1968, XP002108053
- PICKEN P P ET AL: "Inhibition of bovine cathepsin B by amino acid-derived nitriles" BIOCHEM. SOC. TRANS. (BCSTB5,03005127);1990; VOL.18 (2); PP.316, XP002108054 Queen's Univ. Belfast;Med. Biol. Cent.; Belfast; BT9 7BL; UK (GB)

## Description

This invention relates to inhibitors of cysteine proteases, in particular to dipeptide nitrile cathepsin inhibitors and to their pharmaceutical use for the treatment or prophylaxis of diseases or medical conditions in which cathepsins are implicated.

Peptide nitriles are described in BE662788, WO9822433, WO9727200; these compounds are useful for therapeutic use, e.g. as hepatic agents, inhibitors of β-amyloid peptide release, antiviral agents.

The cysteine cathepsins, e.g. cathepsins B, K, L and S, are a class of lysosomal enzymes which are implicated in various disorders including inflammation, rheumatoid arthritis, osteoarthritis, osteoporosis, tumors (especially tumor invasion and tumor metastasis), coronary disease, atherosclerosis (including atherosclerotic plaque rupture and destabilization), autoimmune diseases, respiratory diseases, infectious diseases and immunologically mediated diseases (including transplant rejection).

In accordance with the invention it has been found that dipeptide nitriles are particularly useful as cysteine cathepsin inhibitors and can be used for the treatment of the above-cited cysteine cathepsin dependent conditions.

Accordingly the present invention provides an N-terminal-substituted dipeptide nitrile, i.e. a dipeptide in which the C-terminal carboxy group of the dipeptide is replaced by a nitrile group (-C≡N) and in which the N-terminal nitrogen atom is substituted via a peptide or pseudopeptide linkage which optionally additionally comprises a methylene-hetero atom- linker or an additional hetero atom, directly by aryl, lower alkyl, lower alkenyl, lower alkynyl or heterocyclyl, or a physiologically-acceptable and -cleavable ester or a salt thereof, for use as a pharmaceutical.

The invention further provides a pharmaceutical composition comprising an N-terminal-substituted dipeptide nitrile as defined above as an active ingredient.

The invention also provides a method of treating a patient suffering from or susceptible to a disease or medical condition in which a cathepsin is implicated, comprising administering an effective amount of an N-terminal-substituted dipeptide nitrile as defined above to the patient.

The invention further includes the use of an N-terminal-substituted dipeptide nitrile as defined above for the preparation of a medicament for therapeutic or prophylactic treatment of a disease or medical condition in which a cathepsin is implicated.

The dipeptide nitrile of the invention conveniently comprises α-amino acid residues, including both natural and unnatural α-amino acid residues. Herein the "natural α-amino acid residues" denote the 20 amino acids obtainable by translation of RNA according to the genetic code or the corresponding nitriles thereof, as appropriate. "Unnatural α-amino acid residues" are α-amino acids which have α-substituents other than those found in "natural α-amino acid residues". Preferred α-amino acid residues, as the C-terminal amino acid residue of the dipeptide nitrile, are the nitriles of tryptophan, 2-benzyloxymethyl-2-amino-acetic acid, 2,2-dimethyl-2-amino-acetic acid, 2-butyl-2-amino-acetic acid, methionine, leucine, lysine, alanine, phenylalanine, and glycine and derivatives thereof, e.g. as hereinafter described. Preferred amino acid residues as the N-terminal amino acid residue of the dipeptide nitrile are 1-amino-cyclohaxanecarboxylic acid, 1-amino-cycloheptanecarboxylic acid, phenylalanine, histidine, tryptophan and leucine and derivatives thereof, e.g. as hereinafter described.

Accordingly in preferred embodiments the invention provides compounds according to claim 1. These compounds are hereinafter referred to as Compounds of the invention.

The compounds of the invention exhibit valuable pharmacological properties in mammals and are particularly useful as cysteine cathepsin inhibitors.

The cathepsin inhibitory effects of the compound of the invention can be determined in vitro by measuring the inhibition of e.g. recombinant human cathepsins B, K, L and S. The buffer used in the cathepsin B, L and S assays is a 0.1 M pH 5.8 phosphate buffer containing EDTA (1.33 mM), DTT (2.7 mM) and Brij (0.03%).

The in vitro assays are carried out as follows:
(a) For cathepsin B:
   To a microtiter well is added 100 uL of a 20 uM solution of inhibitor in assay buffer followed by 50 uL of a 6.4 mM solution of Z-Arg-Arg-AMC substrate (Peptides International) in assay buffer. After mixing, 50 uL of a 0.544 nM solution of recombinant human cathepsin B in assay buffer is added to the well, yielding a final inhibitor concentration of 10 uM. Enzyme activity is determined by measuring fluorescence of the liberated aminomethylcoumarin at 440 nM using 380 nM excitation, at 20 minutes. % Enzyme inhibition is determined by comparison of this activity to that of a solution containing no inhibitor. Compounds are subsequently subjected to a dose response curve analysis to determine IC₅₀ values.
(b) For cathepsin K:
   The assay is performed in 96 well microtiter plates at ambient temperature using recombinant human cathepsin K. Inhibition of cathepsin K is assayed at a constant enzyme (0.16 nM) and substrate concentration (54 mM Z-Phe-Arg-MCA - Peptide Institute Inc. Osaka, Japan in 100 mM sodium phosphate buffer, pH 7.0, containing 2 mM dithiothreitol, 20 mM Tween 80 and 1 mM EDTA. Cathepsin K is preincubated with the inhibitors for 30 min, and the reaction is initiated by the addition of substrate. After 30 min incubation the reaction is stopped by the addition of E-64 (2 mM), and fluorescence intensity is read on a multi-well plate reader at excitation and emission wavelengths of 360 and 460 nm, respectively.
(c) For cathepsin L:
   Recombinant human cathepsin L is activated prior to use in this assay: To 500 uL of a 510 nM solution of cathepsin L in a 50 mM pH 5.0 acetate buffer containing 1 mM EDTA, 3 mM DTT and 150 mM NaCl is added 10 uL of a 625 uM solution of dextran sulfate (ave. mw = 8000), and the resulting solution is incubated on ice for 30 min. 4 uL of this solution is then diluted into 46 uL assay buffer, yielding a 40 nM enzyme solution.
   To perform the assay, 100 uL of a 20 uM solution of inhibitor in assay buffer is added to a microtiter well. 50 uL of a 20 uM solution of Z-Phe-Arg-AMC (Peptides International) is then added. After mixing, 50 uL of the activated 40 nM solution of recombinant human Cathepsin L in assay buffer is then added to the well, yielding a final inhibitor concentration of 10 uM. Enzyme activity is determined by measuring fluorescence of the liberated aminomethylcoumarin at 440 nM using 380 nM excitation of 20 minutes. % Enzyme inhibition is determined by comparison of this activity to that of a solution containing no inhibitor. Compounds are subsequently subjected to a dose response curve analysis to determine IC₅₀ values.
(d) For cathepsin S:
   To a microtiter well is added 100 uL of a 20 uM solution of inhibitor is assay buffer. 50 uL of a 700 uM solution of Z-Val-Val-Arg-AMC substrate (Peptides International) is then added. After mixing, 50 uL of a 5.2 nM solution of recombinant human cathepsin S in assay buffer is then added to the well, yielding a final inhibitor concentration of 10 uM. Enzyme activity is determined by measuring fluorescence of the liberated aminomethylcoumarin at 440 nM using 380 nM excitation at 200 minutes. % Enzyme inhibition is determined by comparison of this activity to that of a solution containing no inhibitor. Compounds are subsequently subjected to a dose response curve analysis to determine IC₅₀ values.

In view of their activity as inhibitors of cysteine cathepsin enzymes, Compounds of the Invention are particularly useful in mammals as agents for treatment and prophylaxis of diseases and medical conditions involving elevated levels of cathepsins. Such diseases include diseases involving infection by organisms such as pneumocystis carinii, trypsanoma cruzi, trypsanoma brucei, crithidia fusiculata, as well as parasitic diseases such as schistosomiasis and malaria, tumours (tumour invasion and tumour metastasis), and other diseases such as metachromatic leukodystrophy, muscular dystrophy, amytrophy and similar diseases.

Cathepsins, in particular K, have been implicated in diseases of excessive bone loss, and thus the Compounds of the Invention may be used for treatment and prophylaxis of such diseases, including osteoporosis, gingival diseases such as gingivitis and periodontitis, Paget's disease, hypercalcemia of malignancy, e.g. tumour-induced hypercalcemia and metabolic bone disease. Also the Compounds of the Invention may be use for treatment or prophylaxis of diseases of excessive cartilage or matrix degradation, including osteoarthritis and rheumatoid arthritis as well as certain neoplastic diseases involving expression of high levels of proteolytic enzymes and, matrix degradation.

Compounds of the Invention, are also indicated for preventing or treating coronary disease, atherosclerosis (including atherosclerotic plaque rupture and destabilization), autoimmune diseases, respiratory diseases and immunologically mediated diseases (including transplant rejection).

Compounds of the Invention, in particular cathepsin K selective inhibitor compounds, are particularly indicated for preventing or treating osteoporosis of various genesis (e.g. juvenile, menopausal, post-menopausal, post-traumatic, caused by old age or by cortico-steroid therapy or inactivity).

Beneficial effects are evaluated in in vitro and in vivo pharmacological tests generally known in the art, and as illustrated herein.

The above cited properties are demonstrable in in vitro and in vivo tests, using advantageously mammals, e.g. rats, mice, dogs, or isolated organs and tissues, as well as mammalian enzyme preparations, either natural or prepared by e.g. recombinant technology. Compounds of the Invention can be applied in vitro in the form of solutions, e.g. preferably aqueous solutions or suspensions, and in vivo either enterally or parenterally, advantageously orally, e.g. as a suspension or in aqueous solution, or as a solid capsule formulation. The dosage in vitro may range between about 10⁻⁵ molar and 10⁻⁹ molar concentrations. The dosage in vivo may range, depending on the route of administration, between about 0.1 and 100 mg/kg.

The antiarthritic efficacy of the compounds of the invention for the treatment of rheumatoid arthritis can be determined using models such as or similar to the rat model of adjuvant arthritis, as described previously (R.E. Esser, et. al. J. Rheumatology, 1993, 20, 1176.)

The efficacy of the compounds of the invention for the treatment of osteoarthritis can be determined using models such as or similar to the rabbit partial lateral meniscectomy model, as described previously (Colombo et al. Arth. Rheum. 1993 26, 875-886). The efficacy of the compounds in the model can be quantified using histological scoring methods, as described previously (O'Byme et al. Inflamm Res 1995, 44, S117-S118).

The efficacy of the compounds of the invention for the treament of osteoporosis can be determined using an animal model such as the ovarectomised rat or other similar species in which test compounds are administered to the animal and the presence of markers of bone resorption are measured in urine or serum (e.g. as described in Osteoporos Int (1997) 7:539-543).

The pharmaceutical compositions according to the invention are those suitable for enteral, such as oral or rectal, transdermal, topical, and parenteral administration to mammals, including man, to inhibit cathepsin activity, and for the treatment of cathepsin dependent disorders, in particular inflammation, osteoporosis, rheumatoid arthritis and osteoarthritis, and comprise an effective amount of a pharmacologically active compound of the invention, alone or in combination, with one or more pharmaceutical acceptable carriers.

More particularly, the pharmaceutical compositions comprise an effective cathepsin inhibiting amount of a Compound of the Invention.

The pharmacologically active Compounds of the Invention are useful in the manufacture of pharmaceutical compositions comprising an effective amount thereof in conjunction or admixture with excipients or carriers suitable for either enteral or parenteral application. Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salt and/or polyethyleneglycol; for tablets also c) binders e.g. magnesium aluminum silicate, starch paste, gelatin, tragacanth, methylcellulose, sodium carboxymethylcelculose and or polyvinylpyrrolidone; if desired d) disintegrants, e.g. starches, agar, alginic acid or its sodium salt, or effervescent mixtures; and/or e) absorbents, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1 to 75%, preferably about 1 to 50%, of the active ingredient.

Tablets may be either film coated or enteric coated according to methods known in the art.

Suitable formulations for transdermal application include an effective amount of a compound of the invention with carrier. Advantageous carriers include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin. Matrix transdermal formulations may also be used.

Suitable formulations for topical application, e.g. to the skin and eyes, are preferably aqueous solutions, ointments, creams or gels well-known in the art. Such may contain solubilizers, stabilizers, tonicity enhancing agents, buffers and preservatives.

The pharmaceutical formulations contain an effective cathepsin inhibiting amount of a Compound of the Invention as defined above, either alone or in combination with another therapeutic agent.

In conjunction with another active ingredient, a Compound of the Invention may be administered either simultaneously, before or after the other active ingredient, either separately by the same or different route of administration or together in the same pharmaceutical formulation. The dosage of active compound administered is dependent on the species of warm-blooded animal (mammal), the body weight, age and individual condition, and on the form of administration. A unit dosage for oral administration to a mammal of about 50 to 70 kg may contain between about 5 and 500 mg of the active ingredient.

The present invention also relates to methods of using Compounds of the Invention and their pharmaceutically acceptable salts, or pharmaceutical compositions thereof, in mammals for inhibiting cathepsins, such as cathepsin K, and for the treatment of cathepsin dependent conditions, such as cathepsin K, dependent conditions, described herein, e.g. inflammation, osteoporosis, rheumatoid.arthritis and osteoarthritis.

Particularly the present invention relates to a method of selectively inhibiting cathepsin activity in a mammal which comprises administering to a mammal in need thereof an effective cathepsin inhibiting amount of a Compound of the Invention.

More specifically such relates to a method of treating rheumatoid arthritis, osteoarthritis, and inflammation (and other diseases as identified above) in mammals comprises administering to a mammal in need thereof a correspondingly effective amount of a Compound of the Invention.

The following examples are intended to illustrate the invention and are not to be construed as being limitations thereon. Temperatures are given in degrees Centrigrade. If not mentioned otherwise, all evaporations are performed under reduced pressure, preferably between about 15 and 100 mm Hg (= 20-133 mbar). The structure of final products, intermediates and starting materials is confirmed by standard analytical methods, e.g. microanalysis and spectroscopic characteristics (e.g. MS, IR, NMR). Abbreviations used are those conventional in the art.

The compounds according to examples 3-5, 14-18, 21, 22, 24-31, 33, 35-37, 41, 47, 48, 68-71, 74-77, 79, 81, 83, 86-90, 92-98, 100-104 are hereinafter referred to as reference examples not forming part of the present invention.

### EXAMPLES

### Example 1: Preparation of Indol-4-yl-C(O)-Leu-Gly(CN) of formula X

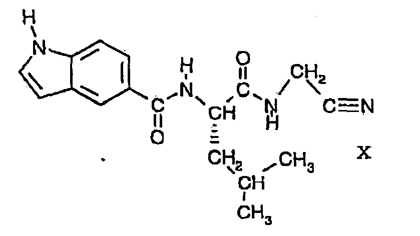

### A. Fmoc-Leu-Gly(CN)

### [1(Cyanomethyl-carbamoyl)-3-methyl-butyl]-carbamic acid 9.H.-fluoren-9-yl methylester

Fmoc-Leucine (0.27mmol) and aminoacetonitrile hydrochloride (32.4mmol) are dissolved in dimethylformamide (300ml) and cooled with ice-salt. HOBt (32.4mmol) and WSCD (32.4mmol) are added, and the reaction mixture is stirred at 4 - 25°C over night. After evaporation of the solvent, the residue is extracted with ethyl acetate. The extract is washed with saturated sodium bicarbonate, 1N hydrochloric acid and brine, dried over magnesium sulfate and the solvent is evaporated. Chromatography on silica gel using n-hexanelethyl acetate=1/1 (v/v) gives the product in 90% yield. mp.173-175°C, Rf=0.68 (chloroform:methanol:acetic acid=90: 10: 1).

### B. H=Leu-Gly(CN)

### 2-Amino-4-methyl-pentanoic acid cyanomethyl-amide

Fmoc-Leu-Gly(CN) (18mmol) is dissolved in 20% piperidine in dimethylformamide (36ml). The reaction mixture is stirred at room temperature for 60min. After evaporation of the solvent and chromatography on silica gel using n-hexane, n-hexane/ethyl acetate=1/1 and 10% methanol in chloroform, the product is obtained in 93% yield.
oil, Rf=0.73 (n-propanol:water:ethyl acetate:ammonia=5:1:2:1).

### C. Indol-5-yl-C(O)-Leu-Gly(CN)

Indol-5-ylcarboxylic acid (1.Oeq.) and H-Leu-Gly(CN) (1.2eq.) are dissolved in dimethylformamide and cooled with ice-salt. HOBt (1.2eq.) and WSCD (1.2eq.) are added and the reaction mixture is stirred at 4 - 25°C over night. After ethyl acetate is added to the reaction mixture, the organic layer is washed with saturated sodium bicarbonate, 1N hydrochloric acid and brine, dried over magnesium sulfate and evaporated. Chromatography on silica gel gives the title product in 70% yield.
mp. 201-204°C, Ref=0.39 (n-hexane:AcOEt-1:2)

### Example 2 5-Amino-quinoline-2-carboxylic acid [1-(cyanomethyl-carbamoyl) -3-methyl-butyl]-amide

5-Nitro-quinoline-2-carboxylic acid [1-(cyanomethyl-carbarnoyl)-3-methyl-butyl)-amide (0.35mmol) is dissolved in tetrahydrofuran (10ml) and methanol (10ml) at room temperature. Na₂S₂0₄ aq * (7mmol) is added to the solution, and the reaction mixture is heated at reflux for 90min. The crude product is isolated by filtration and purified by chromatography on silica gel using 2% methanol in chloroform. The product is obtained in 33% yield. mp.190-194°C, Rf=0.60 (n-hexane:ethyl acetate=1:5). *A.S.Kende et al., Tetrahedron Lett., 25, 923-926, (1984).

### Example 3 p -Acetamidomethylbenzoyl-Leu-GlyCCN) (reference)

p-Aminomethylbenzoyl-Leu-Gly(CN) (see Example 14 below)(0.33mmol) and acetic acid (3.3mmol) are dissolved in dimethylformamide (10ml) and cooled in an ice bath. HOBt (0.4mmol) and WSCD (0.4mmol) are added and the reaction mixture is stirred at 4 - 25°C over night. After evaporation of the solvent, the residue is extracted with ethyl acetate. The extract is washed with saturated sodium bicarbonate, 1N hydrochloric acid and brine, dried over magnesium sulfate and evaporated. Diethylether is added to the residue to give a precipitate, which is collected by filtration and precipitated again from ethyl acetate with diethylether to give the product in 32% yield. mp.176-84.5°C, Ref=0.24 (chloroform-methanol =9:1).

By repeating the procedures described in the above Examples using appropriate starting materials and conditions the following compounds of formula XI are obtained as identified below in Table 1.

**Table 1**

| | | | |
|---|---|---|---|
| 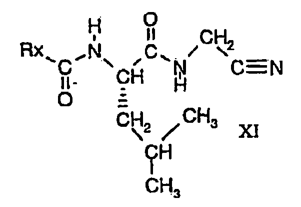 | | | |

| Example No. | Rx | mp. (°C) | Rf (solvent) |
|---|---|---|---|
| 4 (reference) | 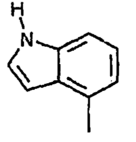 | 52-70 | 0.24 (n-hexane:AcOEt=1:1) |
| 5 (reference) | 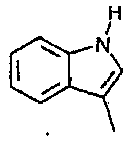 | 150-160 | 0.30 (n-hexane:AcOEt=1:2) |
| 6 | 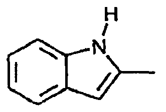 | 170-194 | 0.77 (n-hexane:AcOEt=1:2) |
| 7 | 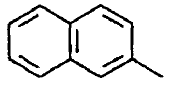 | 169-184.5 | 0.43 (n-hexane:AcOEt=1:1) |
| 8 | 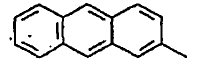 | 210-235.5 | 0.39 (n-hexane:AcOEt=1:1) |
| 9 | 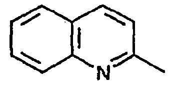 | 174.5- 176.5 | 0.48 (n-hexane:AcOEt=1:2) |
| 10 | 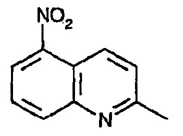 | 163-167 | 0.42 (n-hexane:AcOEt=1:1) |
| 11 | 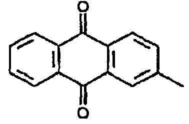 | 234-242 | 0.43 (n-hexane:AcOEt=1:1) |
| 12 | 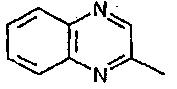 | 156- 158.5 | 0.31 (n-hexane:AcOEt=1:1) |
| 13 | 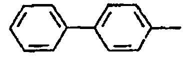 | 191.5- 199 | 0.45 (n-hexane:AcOEt=1:1) |
| 14 (reference) | 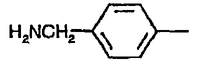 | 57-64 | 0.80 (n-hexane:AcOEt=1:2) |
| 15 (reference) | 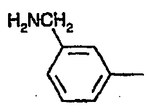 | | 0.31 (chloroform:MeOH=7:3) |
| 16 (reference) | 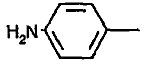 | 89-95 | 0.61 (n-hexane:AcOEt=1:2) |
| 17 (reference) | 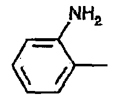 | 223-224 | 0.23 (n-hexane:AcOEt=1:2) |
| 18 (reference) | 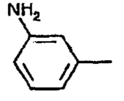 | 143-144 | 0.70 (n-hexane:AcOEt=1:2) |
| 19 | 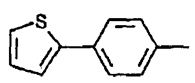 | | 0.33 (n-hexane:AcOEt=1:1) |
| 20 | 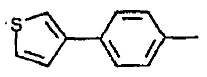 | | 0.47 (n-hexane:AcOEt=1:1) |
| 21 (reference) | 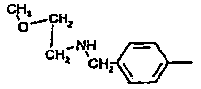 | 122-126 | 0.18 (CH₂Cl₂:MeOH=9:1) |
| 22 (reference) | 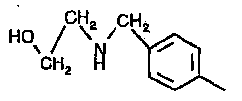 | oil | 0.17 (CH₂Cl₂/MeOH/NH₃=9:1) |
| 23 | 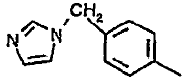 | 248-250 | 0.35 (CH₂Cl₂/MeOH=9:1) |
| 24 (reference) | 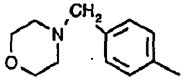 | 136-138 | 0.21 (CH₂Cl₂/MeOH=95:5) |
| 25 (reference) | 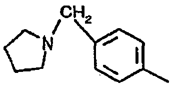 | 225-227 | 0.10 (CH₂Cl₂/MeOH=9:1) |
| 26 (reference) | 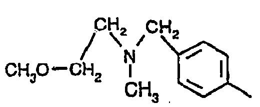 | 97-99 | 0.41 (CH₂Cl₂/MeOH=9:1) |
| 27 (reference) | 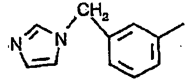 | 164-168 | 0.27 (CH₂Cl₂/lMeOH=9:1) |
| 28 (reference) | 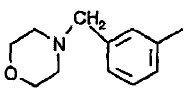 | 114-116 | 0.16 (CH₂Cl₂/MeOH=95:5) |
| 29 (reference) | 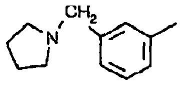 | <70 | 0.16 (CH₂Cl₂/MeOH=9:1) |
| 30 (reference) | 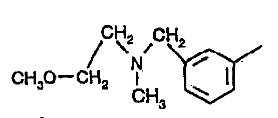 | 89-91 | 0.19 (CH₂Cl₂/MeOH=9:1) |

### Example 31 Indole-2-carboxylic acid {1-[(cyano-dimethyl-methyl)-carbamoyl] -cyclohexyl}-amide (reference)

### A. Fmoc-1-aminocyclohexane carboxylic acid

The title compound is prepared from 1-cyclohexane carboxylic acid (7mmol), Fmoc-CI (7.7mmol) and NaOH (14mmol) in the usual manner in 18 % yield. Ref=0.17 (n-hexane:ethyl acctate=1:2).

### B. Boc-2-Aminoisobutyric acid amide

28% aqueous ammonia (66mmol) is added to the mixed anhydride (prepared from 22mmol of Boc-2-aminobutyric acid and 22mmol of iso-butylcholoroformate by customary procedures) at -20°C. The reaction mixture is stirred at 4 - 25°C overnight. After evaporation of the solvent, the residue is extracted with ethyl acetate. The extract is washed with saturated sodium bicarbonate, 1N hydrochloric acid and brine, dried over magnesium sulfate and evaporated. The crude product is purified by chromatography on silica gel using n-hexane/ethyl acetate=1/1 and n-hexanelethyl acetate=1/2, to give the product in 31 % yield.
mp.168- 177.5°C, Rf=0.41 (chloroform:methanol=9: 1).

### C. 2-Aminobutyric acid amide hydrochloride

Boc-2-Aminoisobutyric acid amide is dissolved in 4N hydrochloride in dioxane. The reaction mixture is stirred at room temperature for 60min. Diethylether is added to the solution to give a white precipitate, which is collected in 91 % yield by filtration. The crude product is used for the next coupling without further purification.
Ref=0.28 (n-PrOH:H₂0:ethyl acetate:NH3=5: 1 :2: 1).

### D. Fmoc-1-Amino-cyclohexanecarboxylic acid (1-carbamoyl-1-methyl-ethyl) -amide

Fmoc-1-aminocyclohexane carboxylic acid (2.2mmol) and 2-aminobutyric acid amide hydrochloride (2.2mmol) are dissolved in dimethylformamide (30ml) and cooled with ice-salt. HOBt (2.6mmol) and WSCD (2.6mmol) are added and the reaction mixture is stirred at 4 - 25°C over night. After evaporation of the solvent, the residue is extracted with ethyl acetate. The extract is washed with saturated sodium bicarbonate, 1N hydrochloric acid and brine, dried over magnesium sulfate and evaporated. The crude product is purified by chromatography on silica gel using n-hexane/ethyl acetate=1/4 and n-hexanelethyl acetate= 1/6, to give the product in quantitative yield.
mp.177.5-1.78.5°C, Ref=0.24 (n-hexane:ethyl acetate=1:5)

### E. Fmoc-1-Amino-cyclohexanecarboxylic acid (cyano-dimethyl-methyl) -amide

Thionyl chloride (2.6mmol) is added to the solution of Fmoc-1-amino-cyclohexanecarboxylic acid (1-carbamoyl-1-methyl-ethyl)-amide (0.86mmol) in dimethylformamide (10ml) at 4°C. The reaction mixture is stirred at 4°C for 2h., ethyl acetate and saturated sodium bicarbonate solution are added and the organic layer is washed with brine, dried over magnesium sulfate and evaporated. The crude product is purified by chromatography on silica gel using n-hexane/ethyl acetate=3/1, to give the product in quantitative yield.
Ref=0.57 (n-hexane:ethyl acetate=1:1).

### F. 1-Amino-cyclohexanecarboxylic acid (cyano-dimethyl-methyl)-amide

Fmoc-1-Amino-cyclohexanecarboxylic acid (cyano-dimethyl-methyl)-amide (2.1 mmol) is dissolved in 20% piperidin in dimethylformamide (6.3ml). The reaction mixture is stirred at room temperature for 60min. After evaporation of the solvent, the crude product is purified by chormatography on silica gel using n-hexane, n-hexane/ethyl acetate=1/1 and 10% methanol in chloroform, to give the product in 31 % yield. oil,
Ref=0.84 (n-propanol:water:ethyl acetate:ammonia=5:1:2:1)

### G. Indole-2-carboxylic acid {1-[(cyano-dimethyl-methyl)-carbamoyl] -cyclohexyl}-amide

2-Indole carboxylic acid (0.51mmol) and 1-amino-cyclohexanecarboxylic acid (cyano-dimethyl-methyl)-amlde (0.61mmol) are dissolved in dimethylformamide (15ml) and cooled in an ice bath. HOBt (0.61 mmol) and WSCD.HCI (0.61mmol) are added and the reaction mixture is stirred at 4 - 25°C over night. After evaporation of the solvent, the residue is extracted with ethyl acetate. The extract is washed with saturated sodium bicarbonate, 1N hydrochloric acid and brine, dried over magnesium sulfate and evaporated. The crude product is purified by chromatography on silica using n-hexane/ethyl acetate=4/1 and n-hexane/ethyl acetate=2/1, to give the product in 71% yield.
mp.200-202°C, Rf=0.55 (n-hexane:ethyl acetate= 1:1)

### Example 32 Synthesis of Naphthalene-2-carboxylic acid [1-(cyanomethyl-carbamoyl) -2-methyl-butyl]-amide

### A. 2-tert-Butyloxycarbonylamino-3-methyl-pentanoic acid cyanomethyl -amide

N-Tertbutyloxycarbonyl-isoleucine semihydrate (3g, 12.5mmol), HOBt (3.71 g, 27.5 mmol, 2.2 eq.) and aminoacetonitrile hydrochloride (1.27g, 13.7mmol, 1.leq.) are dissolved in dimethylformamide (36ml) and WSCD (2.5ml, 13.7mmol, 1.leq.) is added. After stiring for 1 hour at rt, 4% sodium bicarbonate solution is added and the mixture is extracted with ethyl acetate, The organic layer is washed with sodium bicarbonate and dilute hydrochloric acid, dried over magnesium sulfate and evaporated, to give the product in quantitative yield.
mp. 125- 133.5°C, Rf= 0.44 (hexanes:ethyl acetate = 1: 1)

### B. 2-Amino-3-methylpentanoic acid cyanomethyl-amide hydrochloride

2-tert-Butyloxycarbonylamino-3-methyl-pentanoic acid cyanomethyl-amide (2g, 7.4 mmol) is dissolved in 4N hydrochloride in dioxane (10ml). After 15min. at rt the solvent is evaporated to give the product in quantitative yield. The crude product is used for the next step without further purification. Rf (free amine) = 0.33 (ethyl acetate:methanol = 10: 1)

### C. Naphthalene-2-carboxylic acid [1-(cyanomethyl-carbamoyl)-2-methyl -butyl]-amide

2-Naphthoylchloride (255mg, 1.34mmol, 1.1 eq.) is added to the solution of 2-amino-3-methyl-pentanoic acid cyanomethyl-amide hydrochloride (250mg, 1.22mmmo) and triethylamine (0.42ml, 3.04mmol, 2.5 eq.) in 5 ml dichloromethane. After 1 hour at rt 1 N hydrochloric acid is added and the reaction mixture is extracted with ethyl acetate. The organic layer is washed with saturated sodium bicarbonate solution, dried over magnesium sulfate and evaporated. Chromatography on silica gel (hexane/ethyl acetate 10/1 to 5/1, followed by ethyl acetate) gives the product in 97% yield (381mg).
mp. 203.5-207°C, Rf= 0.44 (hexanes:ethyl acetate = 1:1).

### Example 33 Synthesis of Naphtalene-2-carboxylic acid [1-(1-cyano-3-methybutylcarbamoyl)-2-methyl-butyl]-amide (reference)

### A. N-(Naphthalene-2-carbonyl)-isoleucine methylester

L-isoleucine methylester hydrochloride (2.0g, 11.0mmol) and triethylamine (3.1ml, 22.0mmol, 2eq.) are dissolved in dichloromethane (40ml). The solution is cooled in an icebath and 2.-naphthoylchloride (2.1g, 11.0mmol, 1eq.) is added. The reaction mixture is allowed to warm up to rt and after 1 hour 1N hydrochloric acid is added. The mixture is extracted with ethyl acetate, the organic layer is washed with saturated sodium bicarbonate solution, dried over magnesium sulfate and evaporated to give the product in 98% yield.
Rf= 0.50 (hexanes:ethyl acetate = 2: 1).

### B. N-(Naphthalene-2-carbonyl)-isoleucine

N-(Naphthalene-2-carbonyl)-isoleucine methylester (3.14g, 10.5mmol) is stirred in a mixture of methanol (35ml) and 1 N aqueous sodium hydroxide (16.8ml, 1.6 eq.). After 3 hours at rt the mixture is heated for 1 hour at 40°C. 1 N hydrochloric acid and brine is added and the mixture is extracted with ethyl acetate. The organic layer is dried over magnesium sulfate and evaporated to give the product in quantitative yield (partly epimerized).
Rf= 0.32 (hexane:ethyl acetate = 1 :2)

### C. (S)-1-Cyano-3-methyl-butylamine hydrochloride

(S)-N-tert-Butyloxycarbonyl-1-cyano-3-methyl-butylamine (CAS 115654-59-6) (3.7g, 17.4mmol) is dissolved in 4N hydrogenchloride in dioxane (20ml). After 15 minutes at rt the solvent is evaporated, the residue is taken up in diethylether, the solid is filtered and dried in vacuum to give the product in 81 % yield.
Rf (free amine) = 0.34 (hexane:ethyl acetate = 1:1)

### D. Naphthalene-2-carboxylic acid[1-(1-cyano-3-methyl-butylcarbamoyl) -2-methyl-butyl]-amide

N-(Naphthalene-2-carbonyl)-isoleucine (250mg, 0.87mmol), (S)-1-cyano-3-methyl-butylamine (143mg, 0.96mmol. 1.leq.) and HOBt (260mg, 1.93mmol, 2.2eq.) are dissoved in dimethylformamide (5ml) and WSCD (0.17ml, 0.96mmol, 1. 1eq.) is added. After stirring for 1 hour at rt, 4% sodium bicarbonate solution is added and the mixture is extracted with ethyl acetate. The organic layer is washed with sodium bicarbonate and dilute hydrochloric acid, dried over magnesium sulfate and evaporated. Chromatography on silica gel (hexane/ethyl acetate 2/1) gives the product in 68% yield (mixture of epimers).
Rf= 0.43 (hexanes:ethyl acetate = 2: 1)

### Example 34 Synthesis of Naphthalene-2-carboxylic acid [1-(1-cyano-3-methyl -butylcarbamoyl)-3-methyl-butyl]-amide

### A. N-(Naphthalene-2-carbonyl)-leucine

The title compound is prepared analogously is prepared similar to N-(Naphthalene-2 -carbonyl)-isoleucine (see above) in 98% yield, starting from leucine methylester.
Rf= 0.34 (hexanes:ethyl acetate = 1:1)

### B. Naphthalene-2-carboxylic_acid [1-(1-cyano-3-methyl-butylcarbamoyl)-3 -methyl-butyl]-amide

N-(Naphthalene-2-carbonyl)-leucine (250mg, 0.88mmol), (S)-1-cyano-3-methyl-butylamine (143mg, 0.96mmol, 1.1 eq.) and HOBt (260mg, 1.93mmol, 2.2eq.) are dissovled in dimethylformamide (5ml) and WSCD (0.18ml, 0.97 mmol, 1.leq.) is added. After stiring for 1 hour at rt, 4% sodium bicarbonate solution is added and the mixture is extracted with ethyl acetate. The organic layer is washed with sodium bicarbonate and dilute hydrochloric acid, dried over magnesium sulfate and evaporated. Chromatography on silica gel (hexane/ethyl acetate 2/1) gives the product in 79% yield (mixture of epimers).
Rf= 0.44 (hexane:ethyl acetate = 2: 1)

### Example 35 Naphthalene-2-carboxylic acid {1-[1-cyano-2-(1H-indol-3₋yl) -ethylcarbamoyl]-3-methyl-butyl}-amide (reference)

The title compound is prepared analogously to the compound of Example 22. N-(Naphthalene-2-carbonyl)-leucine and 1-cyano-2-(1H-indol-3-yl)-ethylamine (CAS 169545-97-5) are reacted by the same procedure as for Naphthalene-2-carboxylic acid [1-(1-cyano-3-methyl-butylcarbamoyl)-3-methyl-butyl]-amide, to give the product in 36% yield after chromatography on silica gel (hexane/ethyl acetate 1/1) (mixture of epimers).
Rf= 0.59 (hexane:ethyl acetate = 1:1)

### Example 36 Naphthalene-2-carboxylic acid[1-(1-cyano-1-methyl-ethylcarbamoyl)-3-methylbutyl]-amide (reference)

### A. N-tert-Butyloxycarbonyl-1-cyano-1-methyl-ethylamine

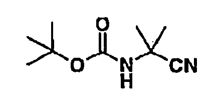

Boc-2-aminoisobutyric acid amide(4.58g, 22.6 mmol) and triethylamine(7 ml, 50mmol, 2.2eq.) are dissolved in THF(100ml) and trifluoroacetic acid anhydride(3.5 ml, 25mmol, 1.1 eq.) is added at 0. The reaction mixture is stirred at 0° for 1 hour.The mixture is concentrated and water is added. The organic layer is extracted with ethyl acetate, washed with brine, dried over sodium sulfate and evaporated. The crude product is purified by chromatography on silica gel using n-hexane/ethyl acetate=20/1, 10/1, 5/1 and 1/1 to give the product in 74 % yield.
Rf=0.45(n-hexare/ethyl acetate=3/1)

### B. 1-Cyano-1-methyl-ethylamine hydrochloride

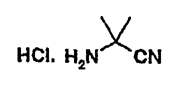

N-tert-Butyloxycarbonyl-1-cyano-1-methyl-ethylamine (3.09 g, 16.8 mmol) is dissolved in dioxane(15 ml) and 4N hydrochloric acid-dioxane (25 ml) is added at 0°. The reaction mixture is stirred at 0° for 1.5 hours, then at rt for 1 hour. The mixture is concentrated and diethyl ether is added. The resulting white precipitate is washed with diethyl ether and dried to give the product in 83 % yield. The crude product is used for the next coupling without further purification.
Rf=0.66(n-PrOH/H₂O/ethyl acetate/NH₃=5/1/2/1)

### C. Naphthalene-2-carboxylic acid[1-(1-cyano-1-methyl-ethylcarbamoyl)-3-methylbutyl]-amide

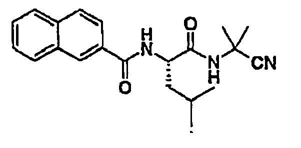

N-(Naphthalene-2-carbonyl)-leucine (279 mg, 0.98 mmol), 1-cyano-1-methyl-ethylamine hydrochloride (137 mg, 1.14 mmol, 1.2 eq.) and HOBt (297 mg, 2.20 mmol, 2.2 eq.) are dissolved in dimethylformamide (5 ml) and WSCD (0.2 ml, 1.09 mmol, 1.1 eq.) is added at -10°. After stirring for 1.5 hours at -10°, 5 % sodium bicarbonate solution is added and the mixture is extracted with ethyl acetate. The organic layer is washed with brine, dried over sodium sulfate and evaporated. Chromatography on silica gel (n-hexane/ethyl acetate=20/1, 10/1, 5/1, 3/1 and 1/1) gives the product in 8.7 % yield (mixture of enantiomers).
Rf=0.54(n-hexane/ethyl acetate=1/1)

### Example 37 Naphthalene-2-carboxylic acid[1-(1-cyano-4-phenyl-propylcarbamoyl)-3-methylbutyl]-amide (reference)

### A. Boc-2-Amino-4-phenyl-butyric acid amide Boc-Hph-CONH2

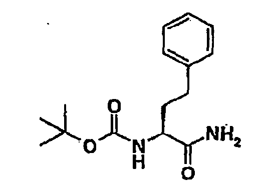

28 % aqueous ammonia (34 mmol) is added to the mixed anhydride (prepared from 16,8 mmol of Boc-homophenylalanine and 17.0 mmol of isobutylchloroformate as usual) at -10 . The reaction mixture is stirred at rt for 4.5 hours. The mixture is concentrated, washed with saturated sodium bicarbonate, 1N hydrochloric acid and brine, dried over sodium sulfate and evaporated to give the product in quantitative yield. The crude product is used for the next reaction without further purification.
Rf=0.60(chloroform/mathanol=10/1)
Thereafter the title compound 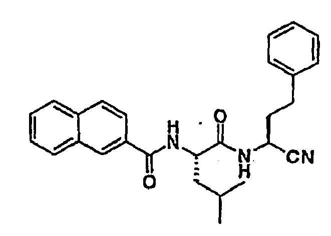 is prepared analogously as in steps A, B and C of Example 36
Rf=0.81(n-hexane/ethyl acetate=1/1)

### Example 38 Naphthalene-2-carboxylic acid[1-(1-cyano-4-phenyl-propylcarbamoyl)-cyclohexyl]-amide

### A. Naphthalene-2-carboxylic acid[(1-methoxycarbonyl)-cyclohexyl]-amide

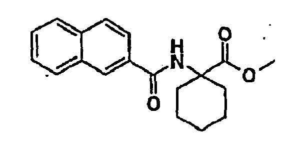

1-Amino-cyclohexanecarboxylic acid methyl ester hydrochloride (1 g, 5.2 mmol) and triethylamine (1.44 ml, 10.3 mmol, 2 eq.) are dissolved in dichloromethane (15 ml) and 2-naphthoyl chloride (1g, 5.2 mmol, 1 eq.) is added at 0°. The reaction mixture is stirred at 0° - 25° for 2 hours and 1N hydrochloric acid is added. The mixture is extracted with ethyl acetate, the organic layer is washed with saturated sodium bicarbonate solution, dried over sodium sulfate and evaporated. Chromatography on silica gel (n-hexane/ethyl acetate=10/1, 5/1, 3/1 and 1/1) gives the product in 93 % yield.
Rf=0.30(n-hexane/ethyl acetate=3/1)

### B. N-(2-Naphthoyl)-1-amino-cyclohexanecarboxylic acid

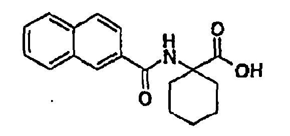

Starting from Naphthalene-2-carboxylic acid[(1-methoxycarbonyl)-cyclohexyl]-amide, the product is prepared analogously to N-(naphthalene-2-carbonyl)-isoleucine in quantitative yield. It is used for the next coupling without further purification.
Rf=0.60(chloroform/methanol=10/1)

### C. Naphthalene:2-carboxylic acid[1-(1-cyano-4-phenyl-propylcarbamoyl)-cyclohexyl]-amide

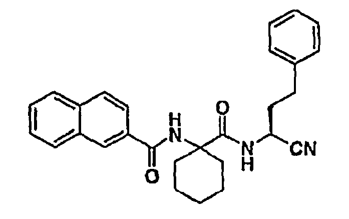

N-(2-Naphthoyl)-1-amino-cyclohexanecarboxylic acid (67 mg, 0.22 mmol), 1-cyano-3-phenyl-propylamine hydrochloride (47 mg, 0.24 mmol, 1.1.eq.) and HOAt (65 mg, 0.48 mmol, 2.2eq.) are dissolved in dimethylformamide (2 ml) and WSCD (0.044 ml, 0.24 mmol, 1.1 eq.) is added at -10° After stirring at 0° - 25° overnight. 5 % sodium bicarbonate solution is added and the mixture is extracted with ethyl acetate. The organic layer is washed with brine, dried over sodium sulfate and evaporated. Chromatography on silica gel(chloroform/acetone=200/1 and 100/1) gives the product in 63 % yield.
Rf=0.73(chloroform/acetone=9/1)

### Example 39 1.H.-Indole-5-carboxylic acid [1-(cyanomethyl-carbamoyl)-cyclohexyl]-amide

1-Amino-cynohexancarboxylic acid cyanomethyl-amide (136mg, 0.50mmol), indol-5-carboxylic acid (80mg, 0.50mmol, 1.0 eq.) and HOBt (74mg, 0.55mmol, 1.1eq.) are dissoved in dimethylformamide (5ml) and WSCD (0.10ml), 0.55mmol, 1.1eq.) is added. After stirring for 20 hour at rt, 4% sodium bicarbonate solution is added and the mixture is extraced with ethyl acetate. The organic layer is washed with sodium bicarbonate, dried over magnesium sulfate and evaporated. Chromatography on silica gel (hexanes/ethyl acetate 2/1, then ethyl acetate) gives the product in 20% yield.
Rf = 0.31 (hexanes/ethyl acetate = 3/1)

### Example 40 Syntheses of N-[1-(cyanometyl-carbamoyl)-cyclohexyl]-4-imidazol-1-ylmethylbenzamide

### A. Boc-1-aminocyclohexane carboxylic acid

The title compound is prepared from 1-cyclohexane carboxylic acid (140 mmol), Boc₂O (154mmol) and Na₂CO₃ (140mmol) in 200 ml dioxane and 100 ml water by conventional methods. Mp. 157-161°C; Rf=0.23 (CH₂Cl₂/MeOH=95:5)

### B. Boc-1-amino-cyclohexanecarboxylic acid (1-(cyanomethyl-carbamoyl)-amide

Boc-1-aminocyclohexane carboxylic acid (40mmol), HOBt (40mmol) and WSCD (42mmol) are dissolved in dimethylformamide (75ml) and stirred for 15 min. at RT. 2-aminoacetonitrile hydrochloride (40mmol) and triethylamine (40mmol) are suspended in DMF (25ml) and added to the reaction mixture which is stirred at 25°C over night. After evaporation of the solvent, the residue is extracted with ethyl acetate. The extract is washed with water, 10% citric acid, brine, sodium bicarbonate, brine and dried over magnesium sulfate and evaporated. The residue is suspended in diethylether and the solid filtered of and dried (vacuum). 7.35g of a white powder with mp.160-162°C, Rf=0.28 (n-hexane:ethyl acetate=1:1) is obtained.

### C. 1-Amino-cyclohexanecarboxylic acid (1-(cyanomethyl-carbamoyl)-amide hydrochloride

HCl in Diethylether (3-4N, 50ml) is added to the solution Boc-1-amino-cyclohexane-carboxylic acid (1-(cyanomethyl-carbamoyl)-amide (33mmol) in THF (50ml) at RT and stirred overnight. The reaction-mixture is cooled with an ice bath to 0-4°C and the solid filtered off and washed with diethylether. The white crystals are dried (vacuum). Mp. 205-209°C; Rf=0.45 (CH₂Cl₂/MeOH=9:1).

### D. N-[1-(cyanomethyl-carbamoyl)-cyclohexyl]-4-bromomethyl-benzamide

4-Bromomethyl-benzoic acid (2.3mmol) is suspended in CH₂Cl₂ (7ml) and cooled to 0-5°C. Chlorenamine (2.3mmol) is added and the mixture is stirred for 45 min. at 0-5°C. 1-Amino-cyclohexanecarboxylic acid (1-(cyanomethyl-carbamoyl) -amide hydrochloride (2.3mmol) and N-ethyldiisopropyl-amine (4.6mmol) in CH₂Cl₂ (7ml) is added at low temperature. The mixture is stirred for 2 hours at 0-5°C and at RT over night. The reaction mixture is diluted with CH₂Cl₂ (40ml), washed with water and dried over magnesium sulfate and evaporated. The residue was suspended in diethylether and the solid filtered of. The crude product is purified by chromatography on silica using CH₂Cl₂/MeOH=97:3. The fractions containing the pure product were collected and evaporated. The residue was suspended in diethylether and the solid filtered of. A white powder with mp.194-196°C, Rf=0.38 (CH₂Cl₂/MeOH=95:5) is obtained.

### E. N-[1-(cyanomethyl-carbamoyl)-cyclohexyl]-4-imidazol-1-ylmethyl-benzamide

N-[1-(cyanomethyl-carbamoyl)-cyclohexyl]-4-bromomethyl-benzamide (0.34mmol) is dissolved in THF (2ml) and sodium-imidazol (0.41 mmol) is added and the reaction mixture stirred at RT over night. After evaporation of the solvent, the residue is extracted with ethyl acetate. The extract is washed with water, dried over magnesium sulfate and evaporated. The residue was suspended in diethylether and the solid filtered of. The crude product is purified by chromatography on silica using CH₂Cl₂/MeOH=9:1. The fractions containing the pure product were collected and evaporated. The residue was suspended in diethylether and the solid filtered of. A white powder with mp.194-196°C; Rf=0.28 (CH₂Cl₂/MeOH=9:1) is obtained.

By repeating the procedure described above in Examples 40, using appropriate starting materials and reaction conditions the following compounds of formula XII are obtained as identified below in Table 2.

**Table 2**

| | | | | |
|---|---|---|---|---|
| 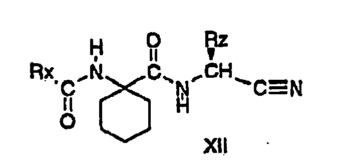 | | | | |

| Example No. | Rx | Rz | mp. (°C) | Rf (solvent) MS(M+1) |
|---|---|---|---|---|
| 41 (reference) | 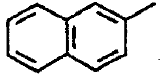 | 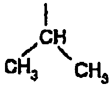 | | 0.26 (hexanes/EtOAc = 3/1) |
| 42 | 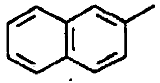 | 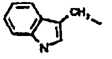 | | 0.50 (hexanes/EtOAc = 1/1) |
| 43 | 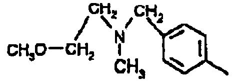 | H | 126-128 | 0.19 (CH₂Cl₂/MeOH=9:1) |
| 44 | 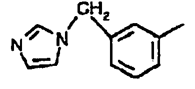 | H | 162-165 | 0.27 (CH₂Cl₂/MeOH=9:1) |
| 45 | 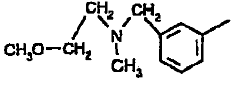 | H | 147-149 | 0.24 (CH₂Cl₂/MeOH=9:1) |
| 46 | 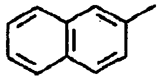 | 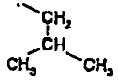 | | 0.26 (hexanes/EtOAc = 3/1) |
| 47 (reference) | " | 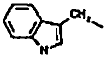 | | 0.50 (hexanes/EtOAc = 1/1) |
| 48 (reference) | 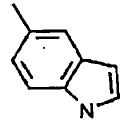 | H | | 0.31 (hexanes/EtOAc = 3/1) |
| 49 | 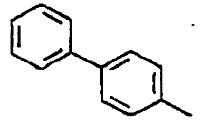 | 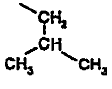 | | 0.31 (n-hexane/EtOAc = 2/1) |
| 50 | 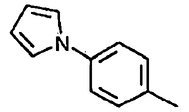 | " | | 0.42 (n-hexane/EtOAc =2/1) |
| 51 | 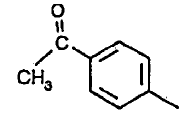 | " | | 0.42 (n-hexane/EtOAc =2/1) |
| 52 | 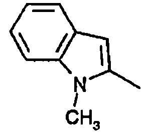 | " | | 0.42 (n-hexane/EtOAc =2/1) |
| 53 | 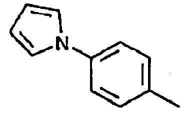 | H | | 0.69 (EtOAc) |
| 54 | 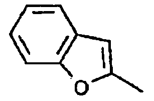 | " | | 0.69 (EtOAc) |
| 55 | 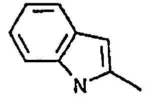 | 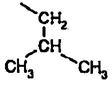 | | 0.58 (n-hexane/EtOAc = 1/1) |
| 56 | 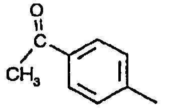 | H | | 0.47 (EtOAc) |
| 57 | 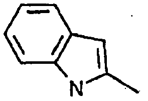 | H | | 0.72 (EtOAc) |
| 58 | 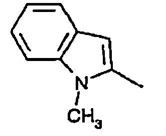 | " | | 0.73 (EtOAc) |
| 59 | 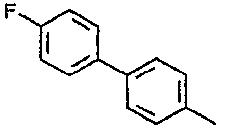 | " | | 0.66(EtOAc) |
| 60 | 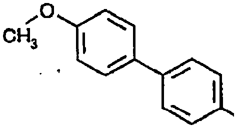 | " | | 0.66 (EtOAc) |
| 61 | 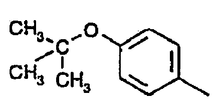 | | | 0.67 (EtOAc) |
| 62 | 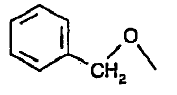 | 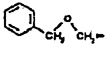 | | 0.24 (toluene/acetone 7/3) 436 |
| 63 | 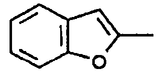 | 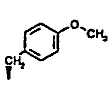 | 1199-201 | 446 |
| 64 | 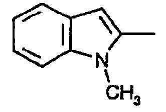 | " | 184-185 | 459 |
| 65 | 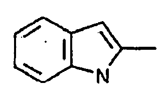 | " | | 0.14(CH₂Cl₂/MeOH 10/0.2) 445 |
| 66 | " | 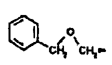 | | 0.54 (petroleum ether/EtOAC 1/1) |
| 67 | 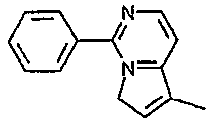 | " | 154-155 | 522 |

### Example 68 Synthesis of N-{1-[(Cyano-dimethyl-methyl)-carbamoyl]-3-methyl-butyl}-4-imidazol-1-ylmethyl-benzamide (reference)

### A. {1-[(Cyano-dimethyl-methyl)-carbamoyl]-3-methyl-butyl}-carbamic acid tert.-butyl ester

Boc-Leu-OH (62mmol), HOBt (62mmol) and WSCD (62mmol) are dissolved in dimethylformamide (150ml) and stirred for 15 min. at RT. 2-Amino-2-methyl-propionamide hydrochloride (62mmol) and triethylamine (62mmol) are suspended in DMF (25ml) and added to the reaction mixture which is stirred at 25°C over night. After evaporation of the solvent, the residue is extracted with ethyl acetate. The extract is washed with water, 10% citric acid, brine, sodium bicarbonate, brine and dried over magnesium sulfate and evaporated. The residue is suspended in diethylether and the solid filtered of and dried (vacuum). 14.78g of a white powder with mp.1.82-184°C, Rf=0.39 (CH₂Cl₂/MeOH=9:1) is obtained.

### B. {1-[(Cyano-dimethyl-methyl)-carbamoyl]-3-methyl-butyl}-carbamic acid .tert.-butyl ester

{1-[(Cyano-dimethyl-methyl)-carbamoyl]-3-methyl-butyl}-carbamic acid .tert.-butyl ester (47mmol) is dissolved in THF (150ml) and cooled to -10°C. Trifluoroacetic acid anhydride (56mmol) and triethylamine (94mmol) are added at -10°C and the stirred mixture is slowly warmed up to 0°C over 2 hours. After evaporation of the solvent, the residue is extracted with ethyl acetate. The extract is washed with water and dried over magnesium sulfate and evaporated. The residue is suspended in diethylether/ pentane and the solid filtered of and dried (vacuum). 9.93g of a white powder with mp.166-168°C, Rf=0.55 (n-hexane:ethyl acetate=1: 1) is obtained.

### C. 2-Amino-4-methyl-pentanoic acid (cyano-dimethyl-methyl)-amide

{1-[(Cyano-dimethyl-methyl)-carbamoyl]-3-methyl-butyl}-carbamic acid .tert.-butyl ester (19mmol) is dissolved in ethyl acetate containing HCl (3-4N, water free) and the mixture is stirred at RT overnight. After evaporation of the solvent, the crude product is purified by chromatography on silica using CH₂Cl₂/MeOH=9:1. The fractions containing the pure product were collected and evaporated. 2.3g of a yellowish oil, Rf=0.36 (CH₂Cl₂/MeOH=9:1) is obtained.

### D. N-{1-[(Cyano-dimethyl-methyl)-carbamoyl]-3-methyl-butyl}-4-bromomethybenzamide

4-Bromomethylbenzoic acid (4.1mmol), HOBt (4.1mmol) and WSCD.HCI (4.1mmol) are dissolved in dimethylformamide (7ml) and stirred for 10 min. 2-Amino-4-methyl-pentanoic acid (cyano-dimethyl-methyl)-amide (4.1mmol) is added in DMF (3ml) and the reaction mixture is stirred at RT overnight. After evaporation of the solvent, the residue is extracted with ethyl acetate. The extract is washed with water, 10% citric acid, brine, sodium bicarbonate, brine and dried over magnesium sulfate and evaporated. The crude product is suspended in diethylether and the solid filtered of and dried (vacuum). A white powder with mp.185-187°C, Rf=0.43 (n-hexane:ethyl acetate=1:1) is obtained.

### E. N-{1-[(Cyano-dimethyl-methyl)-carbamoyl]-3-methyl-butyl}-4-imidazol-1-ylmethyl-benzamide

N-{1-[(Cyano-dimethyl-methyl)-carbamoyl]-3-methyl-butyl}-4-bromomethyl-benzamide (0.18mmol) is dissolved in THF (1ml) and sodium-imidazol (0.41mmol) is added and the reaction mixture stirred at RT over night. After evaporation of the solvent, the residue is extracted with ethyl acetate. The extract is washed with water, dried over magnesium sulfate and evaporated. An oil with Rf=0.44 (CH₂Cl₂/MeOH =9:1) is obtained.

By repeating the procedure described above in Examples 68, using appropriate starting materials and conditions the following compounds of formula XIII are obtained as identified below in Table 3.

**Table 3**

| | | | | |
|---|---|---|---|---|
| 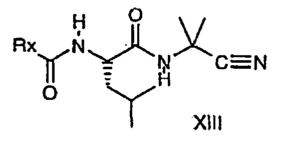 | | | | |

| Example No. | Rx | yield (%) (step B) | mp. (°C) | Rf (solvent) |
|---|---|---|---|---|
| 69 (reference) | 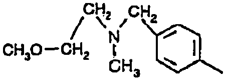 | 58 | 135-137 | 0.29 (CH₂Cl₂/MeOH=9:1) |
| 70 (reference) | 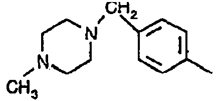 | 51 | 160-162 | 0.16 (CH₂Cl₂/MeOH=9:1) |
| 71 (reference) | 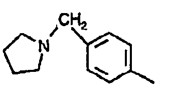 | 44 | 186-188 | 0.23 (CH₂Cl₂/MeOH=9:1) |

### Example 72 N-[1-(Cyanomethyl-carbamoyl)-3-methyl-butyl]-4-(2-pyrrolidin-1-ylethylsulfanyl)-benzamide

### A. 4-(2-Chloroethylsulfanyl)-benzoic acid

4-Mercaptobenzoic acid (65mmol) and 1-Bromo-2-chloro-ethane (71mmol) are dissolved in acetone (120ml) and powdered potassium carbonate (71mmol) is added. The mixture is warmed up to 40°C and stirred for 7 hours. After evaporation of the solvent, the residue is extracted with ethyl acetate. The extract is washed with water and dried over sodium sulfate and evaporated. The crude product is suspended in diethylether and the solid filtered of and dried (vacuum). 7.8g of a white powder with mp. 142-144°C, Ref=0.37 (methylenchlorid/methanol=9/1) is obtained.

### B. 4-(2-Chloroethylsulfanyl)-benzoyl-Leu-Gly(CN)

4-(2-Chloroethylsulfanyl)-benzoic acid (18.5mmol), HOBt (18.5mmol) and WSCD.HCI (19.4mmol) are dissolved in dimethylformamide (50ml) and stirred for 15 min. H-Leu-Gly((CN) (18.5mmol) is added and the reaction mixture is stirred at RT overnight. After evaporation of the solvent, the residue is extracted with ethyl acetate. The extract is washed with water, 10% citric acid, brine, sodium bicarbonate, brine and dried over magnesium sulfate. After evaporation of the solvent, the crude product is purified by chromatography on silica using CH₂Cl₂/MeOH=95:5. The fractions containing the pure product were collected and evaporated. THe product is suspended in diethylether and the solid filtered of and dried (vacuum). 3.15g of a yellowish powder with nip.108-110°C, Rf=0.33 (n-hexane:ethyl acetate=1:1) is obtained.

### C. N-[1-(Cyanomethyl-carbamoyl)-3-methyl-butyl]-4-(2-pyrrolidin-1-ylethylsulfanyl)-benzamide

4-(2-Chloroethylsulfanyl)-benzoyl -Leu-Gly(CN) (1.36mmol) is dissolved DMF (2ml) and pyrrolidine (3mmol) is added. The reaction mixture is stirred for 8 hours at RT, then a catalytic amount of potassium iodide is added and again stirred at 50°C overnight. After evaporation of the solvent, the residue is extracted with ethyl acetate. The extract is washed with water and dried over magnesium sulfate and evaporated. The crude material is applied to a column of silica gel. Elution with CH₂Cl₂/MeOH=93:7 gives the product in 24% yield (Rf=0.12 (CH₂Cl₂/MeOH=95:5).

### Example 73 Synthesis of N-[1-(Cyanomethyl-carbamoyl)-3-methyl-butyl]-4-(2-pyrrolidin-1-ylethylsulfonyl)-benzamide

### A. 4-(2-Chloroethylsulfonyl)-benzoic acid

4-(2-Chloroethylsulfanyl)-benzoic acid (18.4mmol) is suspended in methylene chloride (60ml) and cooled to -10°C. m-Chloroperbenzoic acid (38.6mmol) are added dropwise in methylene chloride (60ml) and the mixture is stirred for e hours at -10°C. The mixture is diluted methylene chloride (100ml) and a 5% solution of sodium thiosulfate in water is added and the mixture vigorously stirred. The mixture is extracted, washed with water and and dried over sodium sulfate and evaporated. The crude product is recrystallized from ethylacetate and the solid filtered of and dried (vacuum). 2.19g of a pale powder with mp. 142-1.44°C, Rf=0.37 (CH₂Cl₂/MeOH=9:1) is obtained.

### B. 4-(2-Chloroethylsulfonyl)-benzoyl-Leu-Gly(CN)

4-(2-Chloroethylsulfonyl)-benzoic acid (8.8mmol), HOBt (8.8mmol) and WSCD.HCI (8.8mmol) are dissolved in dimethylformamide (25ml) and stirred for 15 min. H-Leu-Gly((CN) (18.5mmol) is added and the reaction mixture is stirred at RT overnight. After evaporation of the solvent, the residue is extracted with ethyl acetate. The extract is washed with water, 10% citric acid, brine, sodium bicarbonate, brine and dried over magnesium sulfate. After evaporation of the solvent, the crude product is purified by chromatography on silica using CH₂Cl₂/MeOH=95:5. The fractions containing the pure product were collected and evaporated. The product is suspended in diethylether and the solid filtered of and dried (vacuum). 0.3g of a white powder, Rf=0.25 (CH₂Cl₂/MeOH=95:5) is obtained.

### C. N-[1-(Cyanomethyl-carbamoyl)-3-methyl-butyl]-4-(2-pyrrolidin-1-ylethylsulfonyl)-benzamide

4-(2-Chloroethylsulfonyl)-benzoyl-Leu-Gly(CN) (0.4mmol) is in pyrrolidine (1ml. The reaction mixture is stirred for 1.5 hours at RT. After evaporation of the solvent, the residue is extracted with ethyl acetate. The extract is washed with water and dried over magnesium sulfate and evaporated. The crude material is applied to a column of silica gel. Elution with CH₂Cl₂/MeOH=95:5 gives the product in 43% yield (Rf=0.30 (CH₂Cl₂/MeOH=95:5).

### Example 74 Synthesis of N-[1-(1-Cyano-3-methyl-butylcarbamoyl)-3-methyl-butyl]-4-imidazol-1-ylmethyl-benzamide (reference)

### A. Boc-Leu-Leu-NH2

Boc-Leu-Leu-OH (Bachem, 43.6mmol) is dissolved in THF (250ml) and N-methylmorpholine (43.6mmol) is added. The mixture is cooled to -20°C and isobutyl chloroformate (43.6mmol) is added dropwise. The mixture is stirred for 10 min. and then a 25% aqueous solution of ammonia (52.3mmol) is added at -20°C. The mixture is stirred for 3 hours at -20°C to - 10°C. After evaporation of the solvent, the residue is extracted with ethyl acetate. The extract is washed with water and dried over magnesium sulfate and evaporated. The crude product is suspended in diethylether and the solid filtered of and dried (vacuum). 14.2g of a white powder with mp. 155-156°C, Rf=0.5 (CH₂Cl₂/MeOH=9:1) is obtained.

### B. Boc-Leu-Leu(CN)

Boc-Leu-Leu-NH2 (41mmol) is suspended in THF (200ml) and triethylamine (83mmol) and trifluoroacetic acid anhydride (41mmol) is added at -5°C. The mixture is stirred for 2 hours at -5°C, After evaporation of the solvent, the residue is extracted with ethyl acetate. The extract is washed with water and dried over magnesium sulfate and evaporated. A yellowish oil with Rf=0.59 (n-hexane:ethyl acetate=2:1) is obtained and deprotected without further purification (step C.).

### C. H-Leu-Leu(CN)

Boc-Leu-Leu(CN) (41mmol) is dissolved in THF (50ml) and HCl in diethylether (50ml, 3-4N, water-free) is added at RT and the mixture stirred overnight. After evaporation of the solvent the residue is dissolved in methanol and ammonia in methanol (40ml, 3-4N, water-free) is added and the solid material filtered of. The filtrate is evaporated and the crude product is purified by chromatography on silica using CH₂Cl₂/MeOH=95:5. The fractions containing the pure product were collected and evaporated. 5.07g of a yellowish oil with Rf=0.43 (CH₂Cl₂/MeOH=9:1) is obtained.

### D. 4-Bromomethylbenzoyl-Leu-Leu(CN)

4-Bromomethylbenzoic acid (6.67mmol), HOBt (6.67mmol) and WSCD.HCI (7.0mmol) are dissolved in dimethylformamide (15ml) and stirred for 15 min. H-Leu-Leu(CN) (6.67mmol) is added and the reaction mixture is stirred for 2.5 hours at RT. After evaporation of the solvent, the residue is extracted with ethyl acetate. The extract is washed with water, 10% citric acid, brine, sodium bicarbonate, brine and dried over magnesium sulfate. After evaporation of the solvent, the crude product is purified by chromatography on silica using CH₂Cl₂/MeOH=97:3. The fractions containing the pure product were collected and evaporated. 1.74g of a yellowish oil with Rf=0,59 (CH₂Cl₂/MeOH=95:5) is obtained.

### E. N-[1-(1-Cyano-3-methyl-butylcarbamoyl)-3-methyl-butyl]-4-imidazol-1-ylmethylbenzamide

4-Bromomethylbenzoyl-Leu-Leu(CN) (1.23mmol) is dissolved in THF (5ml) and sodium-imidazol (1.48mmol) is added and the reaction mixture stirred at RT over night. After evaporation of the solvent, the residue is extracted with ethyl acetate. The extract is washed with water, dried over magnesium sulfate and evaporated. The crude product is purified by chromatography on silica using CH₂Cl₂/MeOH=95:5. The fractions containing the pure product were collected and evaporated. The product is suspended in diethylether and the solid filtered of and dried (vacuum). A white powder with mp.100-103°C, Rf=0.36 (CH₂Cl₂/MeOH=9:1) is obtained.

By repeating the procedure described above in Example 74, using appropriate starting materials and conditions the following compounds of formula XIV are obtained as identified below in Table 4.

**Table 4**

| | | | | |
|---|---|---|---|---|
| 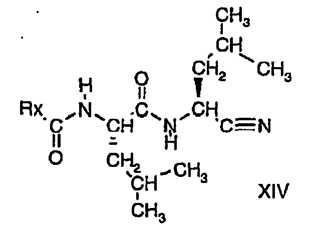 | | | | |

| Example No. | Rx | yield (%) (step B) | mp. (°C) | Rf (solvent) |
|---|---|---|---|---|
| 75 (reference) | 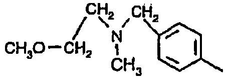 | 45 | - | 0.17 (CH₂Cl₂/MeOH=95:5) |
| 76 (reference) | 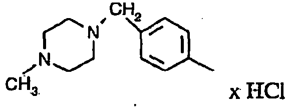 | 51 | - | 0.23 (CH₂Cl₂/MeOH=9:1) |
| 77 (reference) | 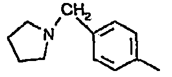 | 64 | - | 0.31 (CH₂Cl₂/MeOH=9:1) |

### Example 78 [1-(2-Benzyloxy-1-cyano-ethylcarbamoyl)-3-methyl-butyl]-carbamic acid benzyl ester

### A. 3-Benzyloxy-2-(2-benzyloxycarbonylamino-4-methyl-pentanoylamino)-propionic acid

To a suspension of 0.975 g H-Ser(OBzl)-OH in 5 ml of methylene chloride is added 1.52 ml of trimethylchlorosilane. After ten minutes at room temperature 0.98 ml of N,N-diisopropylethyl amine and 1.81g of benzyloxy leucine N-hydroxysuccinimidester is added. The reaction mixture is stirred for 2 hours at room temperature and diluted with ethyl acetate. Ethyl acetate is washed once with saturated NH₄Cl-solution and once with H₂O, then dried over sodium sulfate, the solvent is removed and the residue is crystallized from diethylether.
¹H-NMR (CDCl₃, ppm): 7.30 (m, 10H), 6.83 (d, 1H), 5.32 (d, 1H), 5.10 (s, 2H), 4.71 (m, 1H), 4.50 (s, 2H), 4.28 (m, 1H), 3.92 (m, 1H), 3.67 (m, 1H), 1.46 -1.79 (m, 3H), 0.92 (d, 6H).

### B. [1-(2-Benzyloxy-1-carbamoyl-ethylcarbamoyl)-3-methyl-butyl]-carbamic acid benzyl ester

To a solution of 0.980 g of 3-benzyloxy-2-(2-benzyloxycarbonylamino-4-methyl-pentanoylamino)-propionic acid and 0.25 ml of N-methylmorpholine in 12 ml of tetrahydrofuran 0.3 ml of isobutylchloroformate is added dropwise at -15°C. The reaction mixture is stirred at -15°C for 10 minutes, then 4 ml of aqueous NH₃ (25%) is added dropwise over a time period of 5 minutes. The reaction mixture is stirred for additional 15 minutes and diluted with ethyl acetate. Ethyl acetate is washed once with saturated NH₄Cl-solution and once with H₂O, then dried sodium sulfate, the solvent is removed und the residue is triturated with diethylether.
¹H-NMR (CDCl₃, ppm): 7.38 (m, 10H), 6.87 (d, 1H), 6.60 (m (br.), 1H), 5.41 (m (br.), 1H), 5.12 (d, 1H), 5.08 (s, 2H), 4.50 (d, 2H), 4.20 - 3.92 (m, 2H), 3.50 (m, 1H), 1.70 - 1.41 (m, 3H), 0.90 (d, 6H).

### C. [1-(2-Benzyloxy-1-cyano-ethylcarbamoyl)-3-methyl-butyl]-carbamic acid benzyl ester

0.3 ml of trifluoroacetic acid anhydride is added dropwise to a solution of 0.9 g of [1-(2-benzyloxy-1-carbamoyl-ethylcarbamoyl)-3-methyl-butyl]-carbamic acid benzyl ester and 0.6 ml of triethylamine in 15 ml of tetrahydrofuran at -5°C. The reaction mixture is stirred at -5°C for 3 hours and then for 12 hours at room temperature. The reaction mixture is then poured into H₂O and the aqueous layer is extracted three times with ethyl acetate. The combined organic layers are washed once with H₂O and once with brine, then dried over sodium sulfate, the solvent is removed and the residue is crystallized from diethylether/ hexane. Mp.: 126 -127°C.

The following compounds formula XV, as identified in Table 5 below, are prepared analogously to the compound of Example 78.

**Table 5**

| | | |
|---|---|---|
| 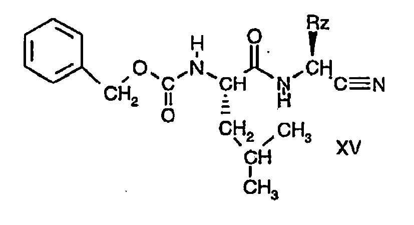 | | |

| Example No. | Rz | mp. (°C) MS (M+1) |
|---|---|---|
| 79 (reference) | 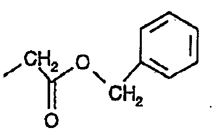 | 100-101° |
| 80 | 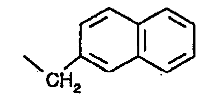 | 157-158° |
| 81 (reference) | 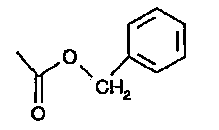 | 157-158° |
| 82 | 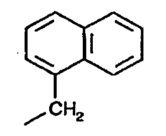 | 159-161° |
| 83 (reference) | 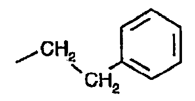 | 106-107° |
| 84 | 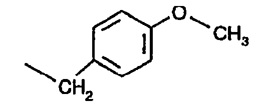 | 126-127° 424 |
| 85 | 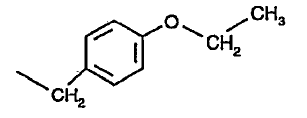 | 144-146 438 |

### Example 86 2-[2-(4-Chloro-phenylamino)-acetylamino]-4-methyl-pentanoic acid cyanomethyl-amide (reference)

(4-Chloro-phenylamino)-acetic acid (0.5 g) and H-Leu-Gly((CN).HCl (0.55 g) are dissolved in dimethylformamide (4 ml). HOBt (0.44 g), WSCD.HCl (0.54 g), triethylamine (0.37 ml) is added and the reaction mixture is stirred for 18 hours. After evaporation of the solvent, the residue is extracted with ethyl acetate. The extract is washed with 10% citric acid, brine, sodium bicarbonate, brine and dried over magnesium sulfate and evaporated. The crude product is slurried in diethylether and the solid filtered of and dried (vacuum) yielding a white powder with mp.131-134°C.

By repeating the procedure described above in Example 86, using appropriate starting materilas and conditions the following compounds of formula XVI are obtained as identified below in Table 6.

**Table 6**

| | | | |
|---|---|---|---|
| 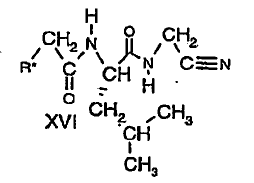 | | | |

| Example No. | R* | mp. (°C) | Rf (solvent) |
|---|---|---|---|
| 87 (reference) | 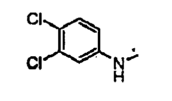 | foam | 0.32 (CH2Cl2/MeOH= 95:5) |
| 88 (reference) | 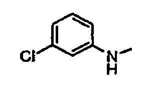 | 110-115 | |
| 89 (reference) | 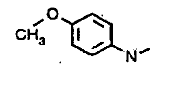 | foam | 0.30 (CH2Cl2/MeOH= 95:5) |
| 90 (reference) | 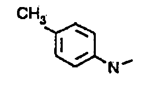 | 130-133 | |
| 91 | 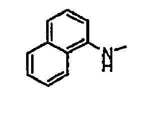 | amorph | 0.42 (CH2Cl2/MeOH= 95:5) |
| 92 (reference) | 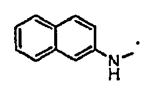 | 131-133 | |
| 93 (reference) | 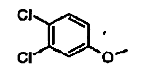 | 108-110 | |
| 94 (reference) | 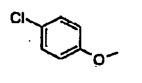 | Resin | 0.43 (CH2Cl2/MeOH= 95:5) |
| 95 (reference) | 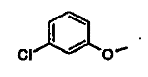 | 77-79 | |
| 96 (reference) | 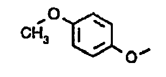 | Oil | |
| 97 (reference) | 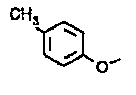 | Resin | 0.53 (CH2Cl2/MeOH= 95:5) |
| 98 (reference) | 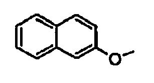 | foam | 0.47 (CH2Cl2/MeOH= 95:5) |
| 99 | 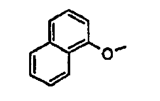 | 143-146 | |
| 100 (reference) | 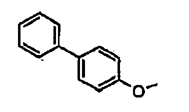 | 119-121 | |
| 101 (reference) | 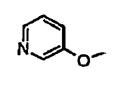 | Resin | 0.26 (CH2Cl2/MeOH= 95:5) |
| 102 (reference) | 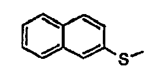 | 146-149 | |

By repeating the procedures described in the above Examples using appropriate starting materials and reaction conditions the following compounds of formula XVII are obtained as identified below in Table 7.

**Table 7**

| | | | | | |
|---|---|---|---|---|---|
| 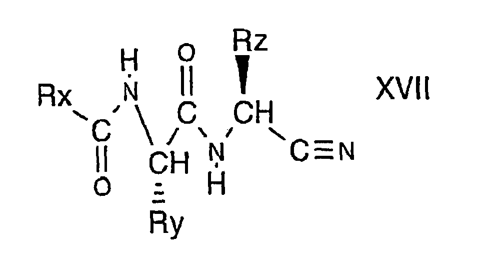 | | | | | |

| Example No. | Rx | Ry | Rz | mp. (°C) | Rf (solvent)/MS (M+1) |
|---|---|---|---|---|---|
| 103 (reference) | 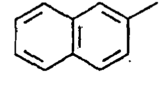 | 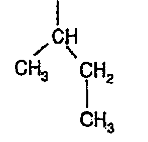 | H | 203.5-207 | 0.44 (hexanes/EtOAc = 1/1) |
| 104 (reference) | " | 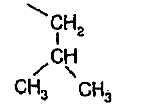 | 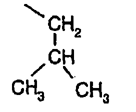 | | 0.44 (hexanes/EtOAc = 2/1) |
| 105 | 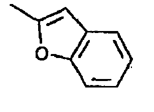 | " | 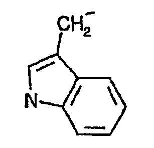 | | 0.52(n-hexane/EtOAc =1/1) |
| 106 | 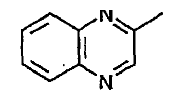 | " | " | | 0.45(n-hexane/EtOAc =1/1) |
| 107 | 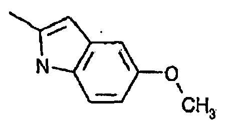 | " | 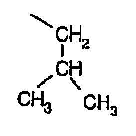 | | 0.30(n-hexane/EtOAc =3/1) |
| 108 | 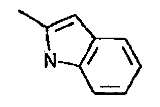 | " | " | | 0.40(n-hexane/EtOAc =2/1) |
| 109 | " | " | 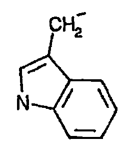 | | 0.24(n-hexane/EtOAc =4/3) |
| 110 | 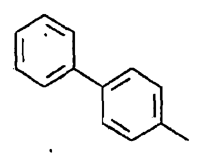 | " | 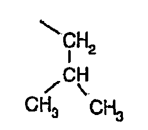 | | 0.36(n-hexane/EtOAc =2/1) |
| 111 | 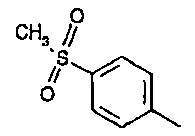 | " | " | | 0-17 (n-hexane/EtOAc = 1/1) |
| 112 | 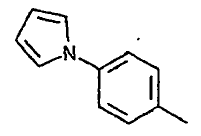 | " | " | | 0.27 (n-hexane/EtOAc = 1/1) |
| 113 | 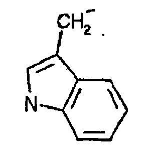 | " | H | | 0.45 (EtOAc) |
| 114 | 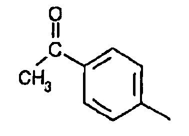 | " | " | | 0.58 (EtOAc) |
| 115 | 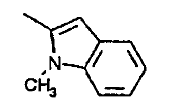 | " | " | | 0.28 (n-hexane/EtOAc = 1/1) |
| 116 | 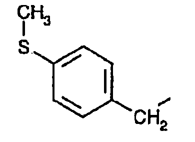 | " | 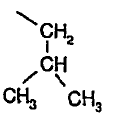 | | 0.46 (n-hexane/EtOAc = 1/1) |
| 117 | 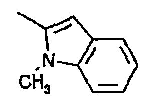 | " | " | | 0.41 (n-hexane/EtOAc=2/1) |
| 118 | 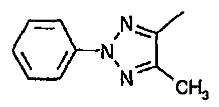 | " | H | | 0.79 (EtOAc) |
| 119 | 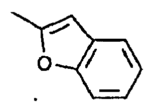 | " | " | | 0.74 (EtOAc) |
| 120 | 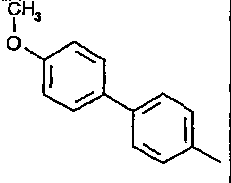 | " | 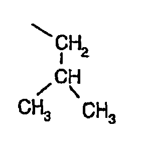 | | 0.52 (n-hexane/EtOAc= 1/1) |
| 121 | 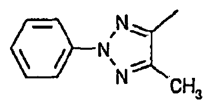 | " | " | | 0.60 (n-hexaneBtOAc= 1/1) |
| 122 | 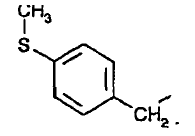 | " | H | | 0.60 (EtOAc) |
| 123 | 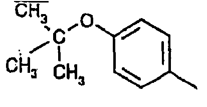 | " | 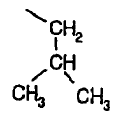 | | 0.54 (n-hexane/EtOAc= 1/1) |
| 124 | u | " | H | | 0.26 (n-hexane/EtOAc = 1/1) |
| 125 | 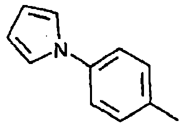 | " | 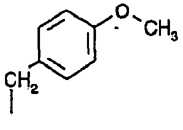 | | 0.41 (n-hexane/EtOAc = 1/1) |
| 126 | 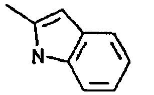 | " | " | | 0.43 (n-hexane/EtOAc= 1/1) |
| 127 | " | " | 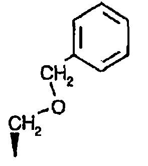 | 151-152 | 433 |
| 128 | 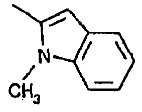 | " | " | 137-138 | 447 |
| 129 | 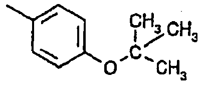 | " | " | | 0.65 (toluene/acetone 7/3) 466 |
| 130 | 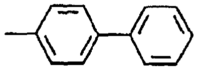 | " | " | 163-165 | 470 |
| 131 | 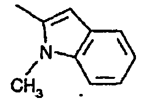 | " | 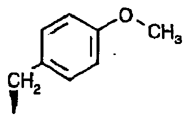 | 214-215 | 447 |
| 132 | 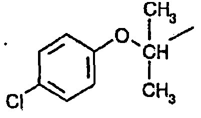 | " | " | | 0.66 (CH₂Cl₂/CH₃OH 10/0.5) 487 |
| 133 | 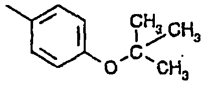 | " | " | 176-178 | 466 |
| 134 | 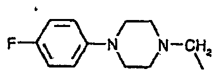 | " | " | 106-110 | 522 |
| 135 | 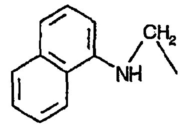 | | " | | 0.46 (CH₂Cl₂/CH₃OH 10/0.5) 477 |
| 136 | 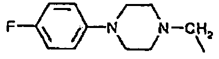 | " | " | | 0.51 (CH₂Cl₂/CH₃OH 10/0.5) 510 |
| 137 | 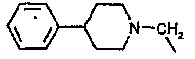 | " | " | | 0.26 (CH₂Cl₂/CH₃OH 10/0.5) 491 |
| 138 | 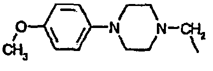 | " | 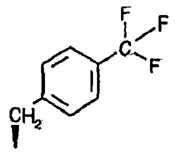 | 121-126 | 560 |
| 139 | 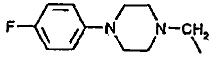 | " | " | 141-143 | 548 |
| 140 | 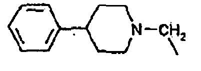 | " | " | 233-234 | 529 |
| 141 | 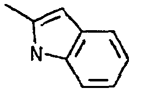 | " | " | 199-202 | 471 |
| 142 | 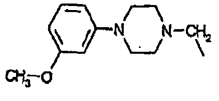 | " | 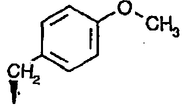 | 122-126 | 522 |
| 143 | 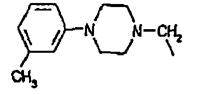 | " | " | | 0.40 (CH₂Cl₂/CH₃OH 10/0.5) 506 |
| 144 | 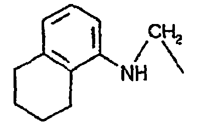 | " | 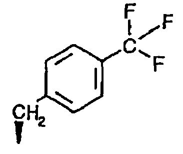 | 131-133 | 515 |
| 145 | 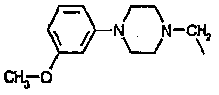 | " | | 114-115 | 560 |
| 146 | 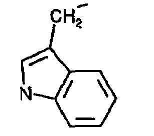 | " | " | 180-182 | 485 |
| 147 | " | " | 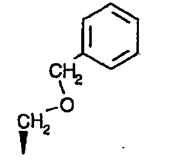 | 129-133 | 447 |
| 148 | 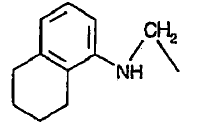 | " | " | | 0.50 (toluene/EtOH 9/1) 477 |
| 149 | 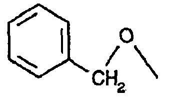 | " | 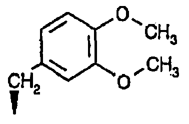 | 145-146 | 454 |
| 150 | " | " | 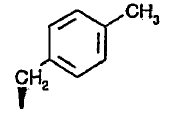 | 152-153 | 408 |
| 151 | 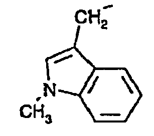 | " | 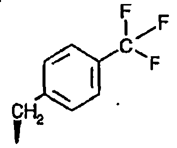 | 210-211 | 499 |
| 152 | 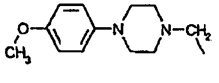 | " | 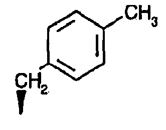 | 148-149 | 506 |
| 153 | 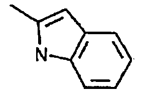 | " | " | 236-237 | 417 |

Compounds of Examples 1 to 153 are typically selective inhibitors of cathepsin K and generally have IC₅₀s for inhibition of human cathepsin K of from about 100 to about 1 nM or less, e.g. about 0.5nM.

Representative compounds e.g. as described in the the above Examples typically have IC₅₀s for inhibition of Cathepsin K in the range from less than 1 up to about 100 nm, and IC₅₀s for inhibition of Cathepsin K which are at least 10 to about 1000 times less than their IC₅₀s for inhibition of Cathepsin L and Cathepsin S, e.g. when tested in the assays described above.

The cathepsin K selective compounds of the invention are particularly indicated for preventing or treating osteoporosis of various genesis (e.g. juvenile, menopausal, post-menopausal, post-traumatic, caused by old age or by cortico-steroid therapy or inactivity).

### Example 420 Preparation of 1,000 capsules each containing 25 mg of a Compound of the Invention, using the following ingredients:

| | |
|---|---|
| Compound of the Invention | 25.00 g |
| Lactose | 192.00 g |
| Modified starch | 80.00 g |
| Magnesium stearate | 3.00 g |

Procedure: All the powders are passed through a screen with openings of 0.6 mm. Then the drug substance is placed in a suitable mixer and mixed first with the magnesium stearate, then with the lactose and starch until homogeneous. No. 2 hard gelatin capsules are filled with 300 mg of said mixture each, using a capsule filling machine.

## Claims

1. A compound selected from the following:

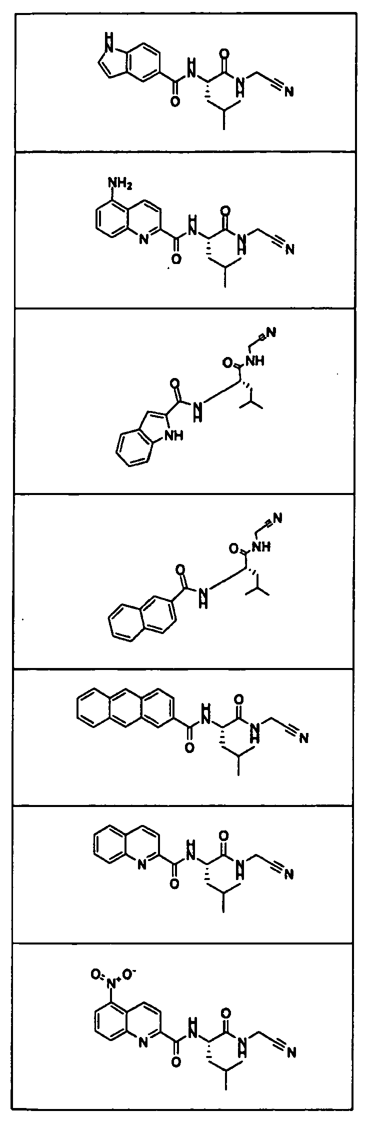

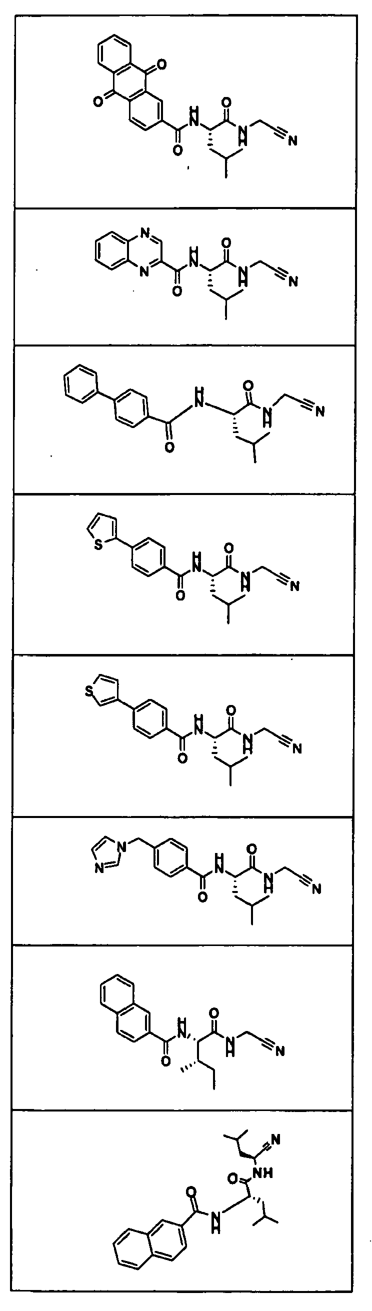

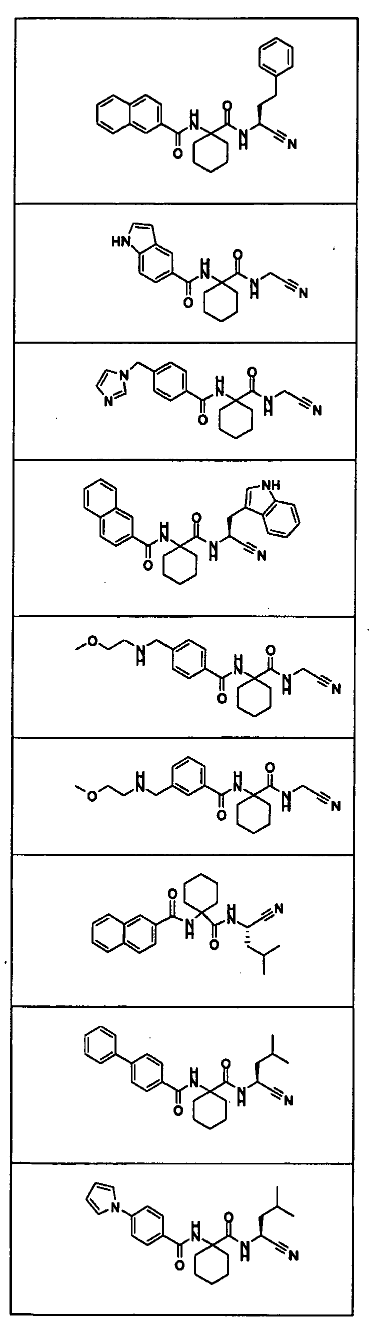

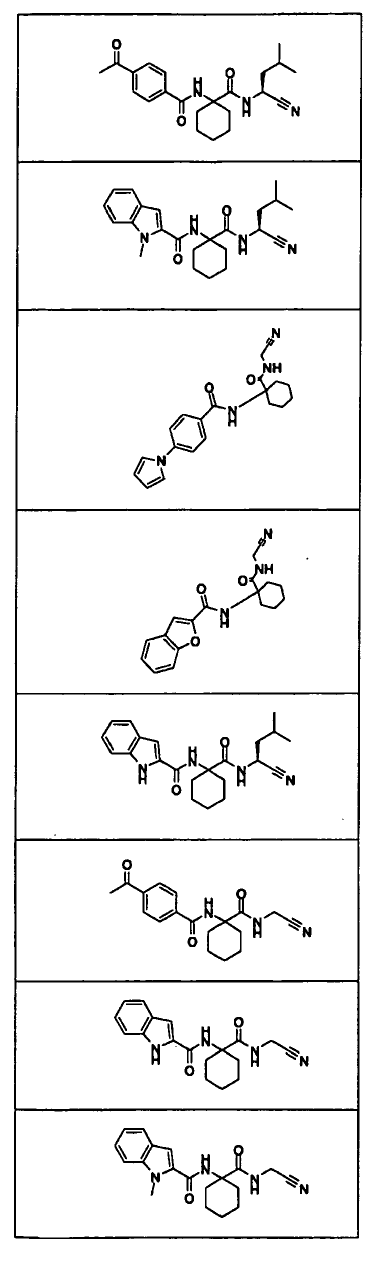

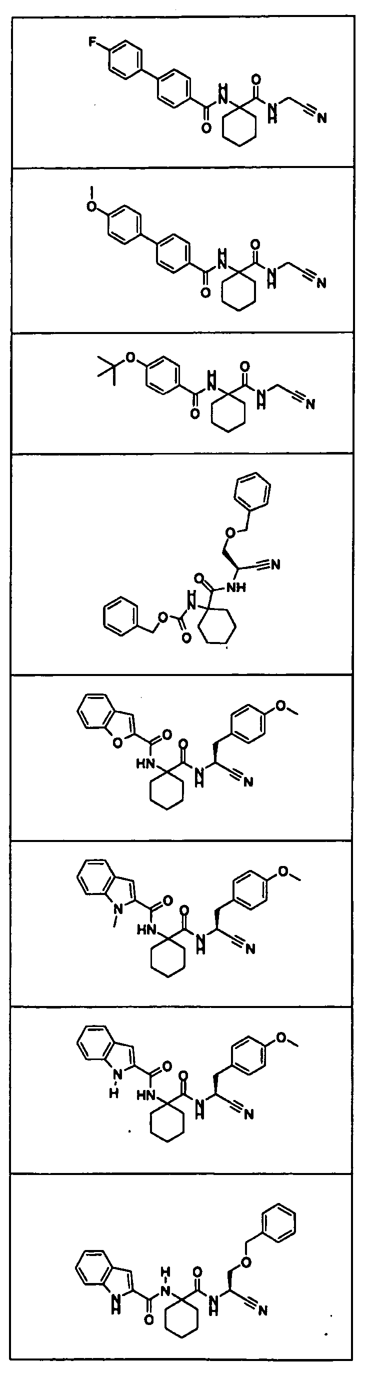

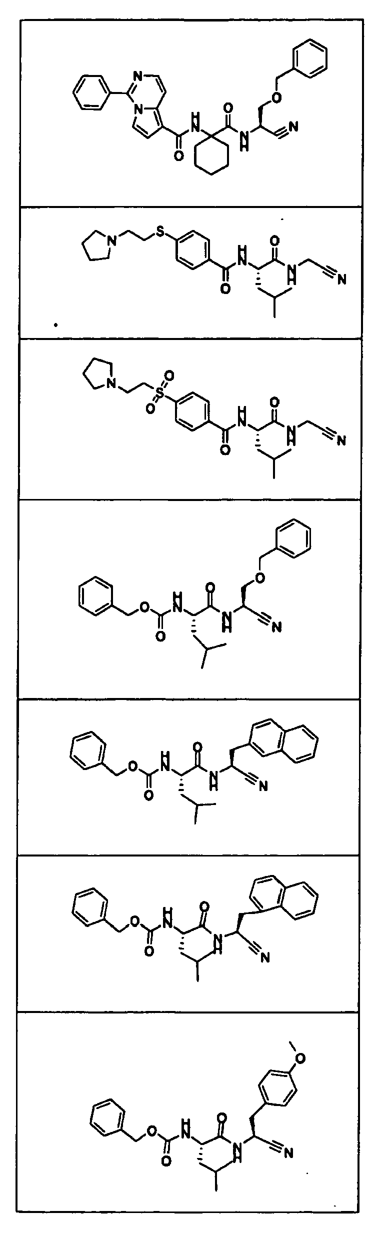

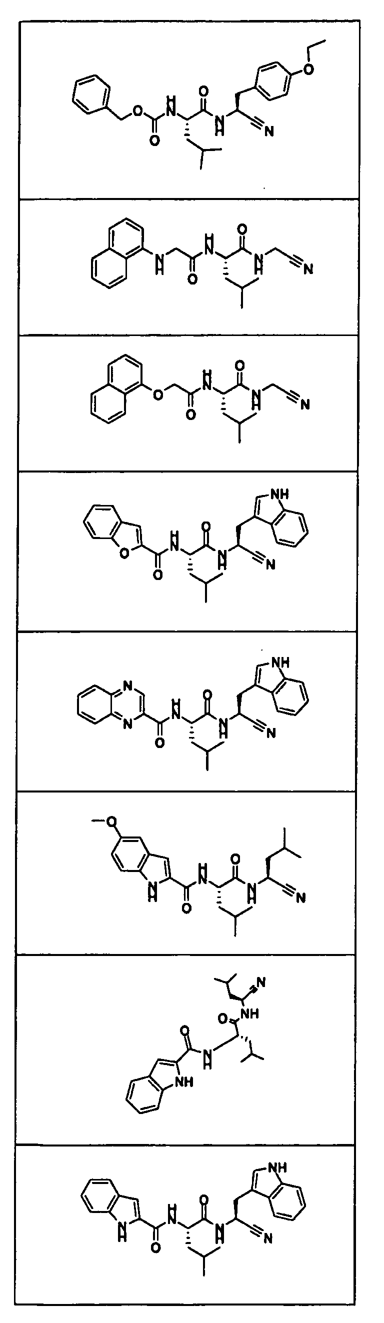

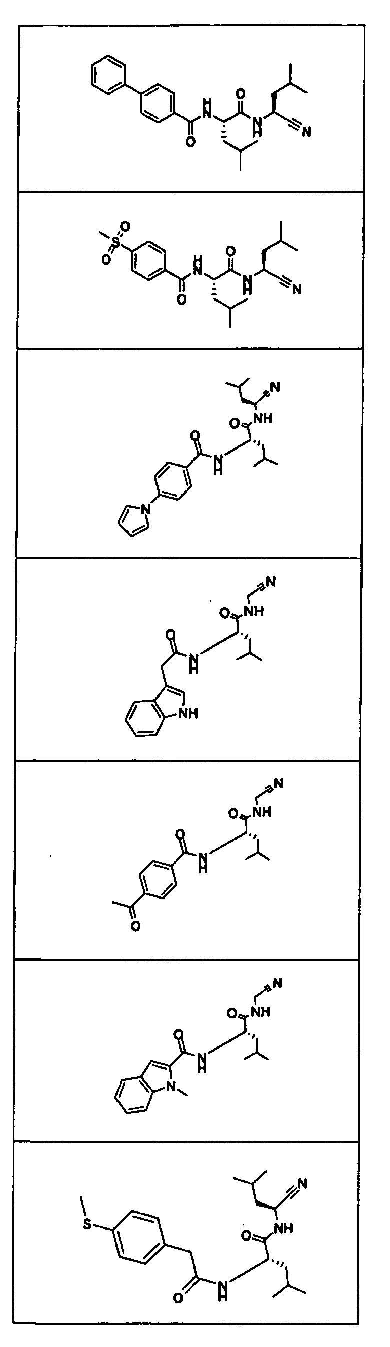

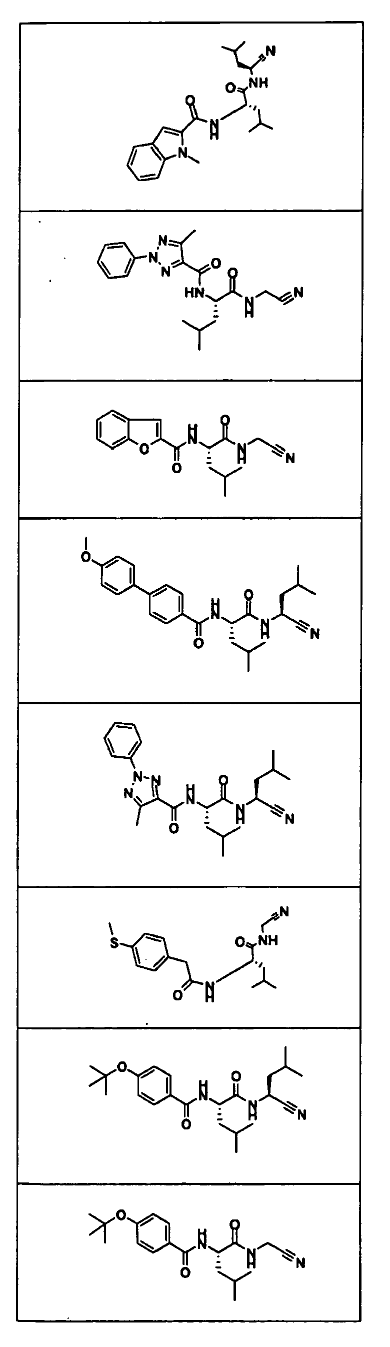

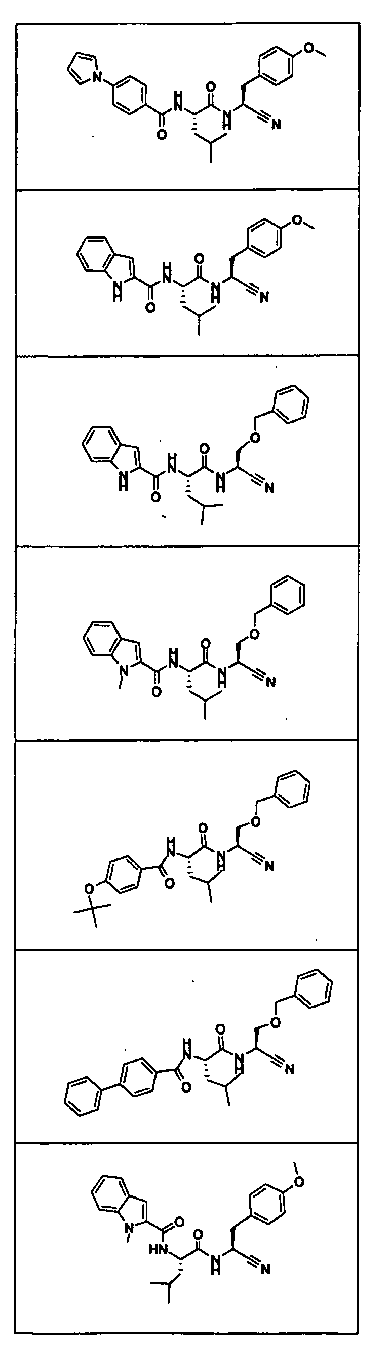

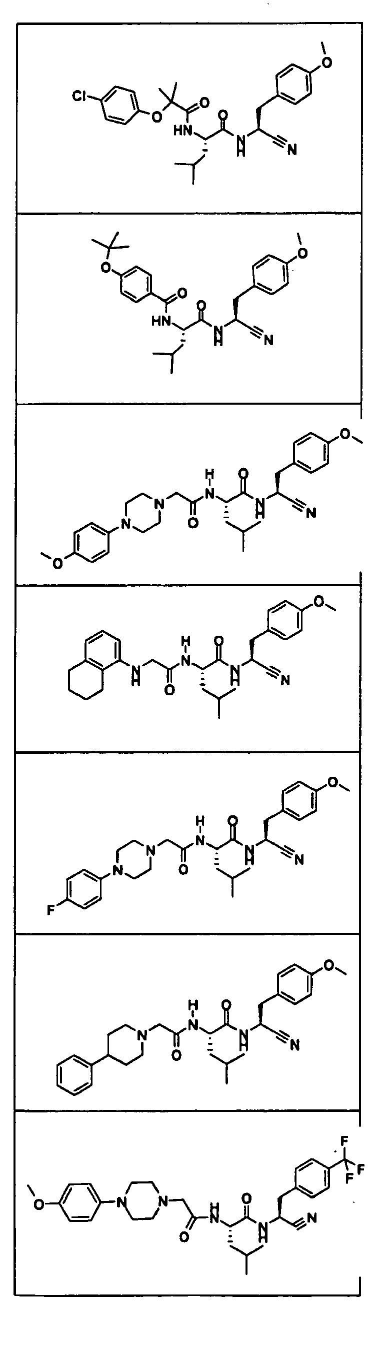

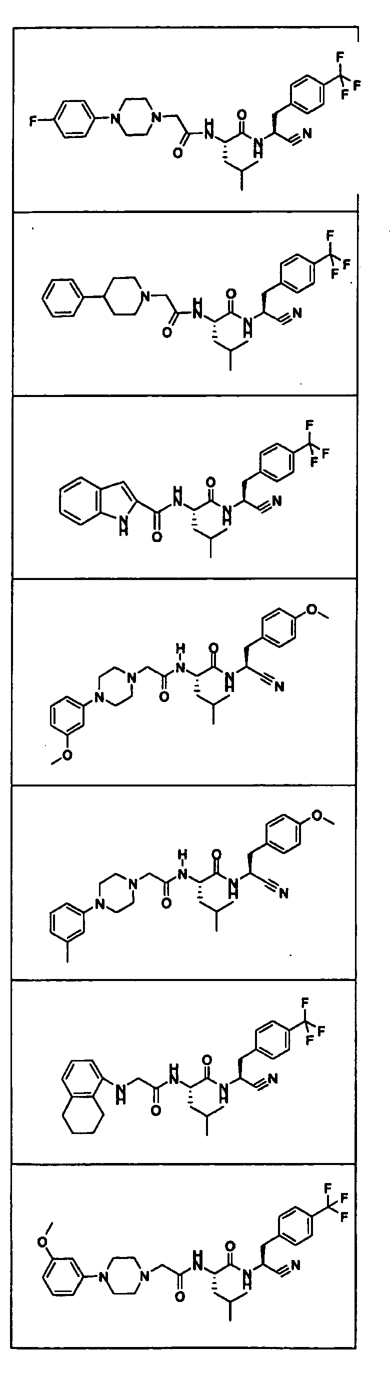

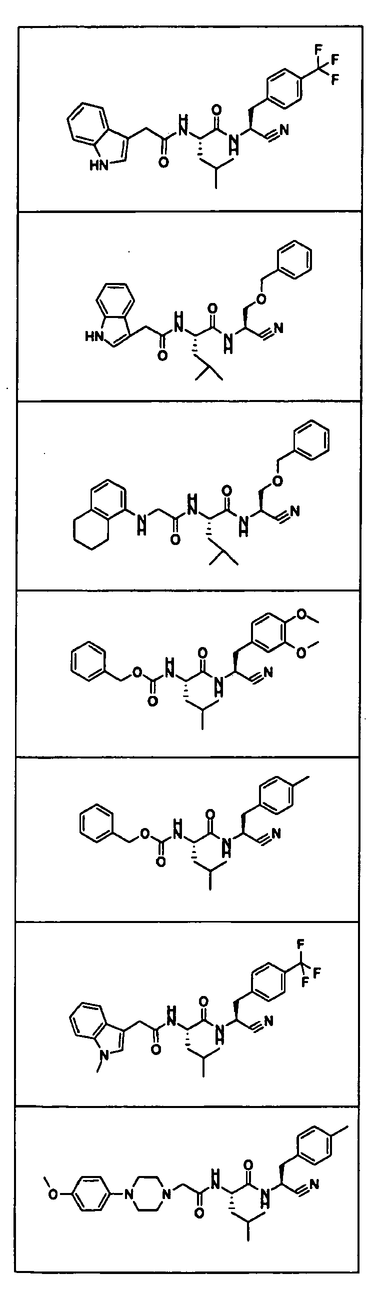

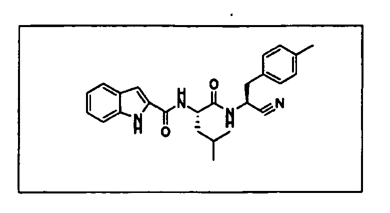

2. A compound according to claim 1 for use as a medicament.

3. Use of a compound according to claim 1 for the preparation of a medicament for preventing or treating osteoporosis.

4. A pharmaceutical composition comprising a compound according to claim 1.

## Patentansprüche

1. Verbindung, die aus den folgenden ausgewählt ist:

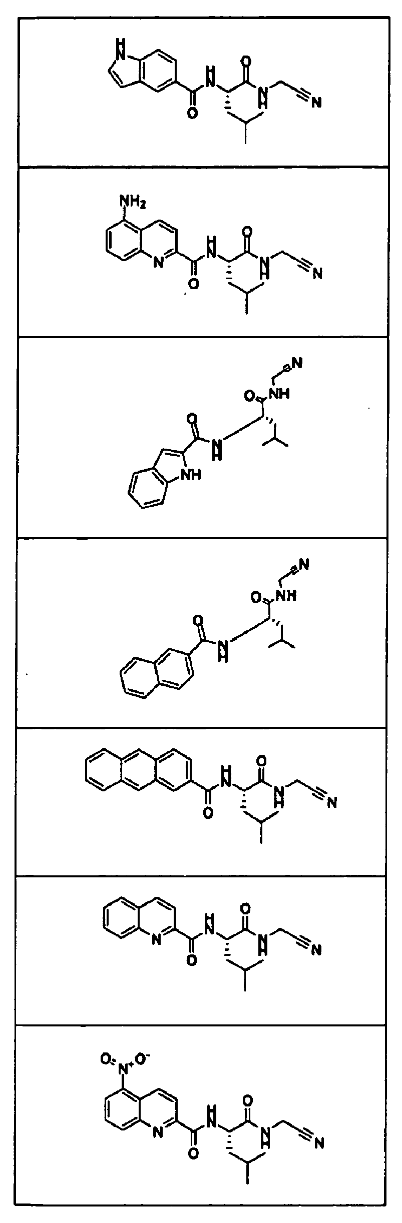

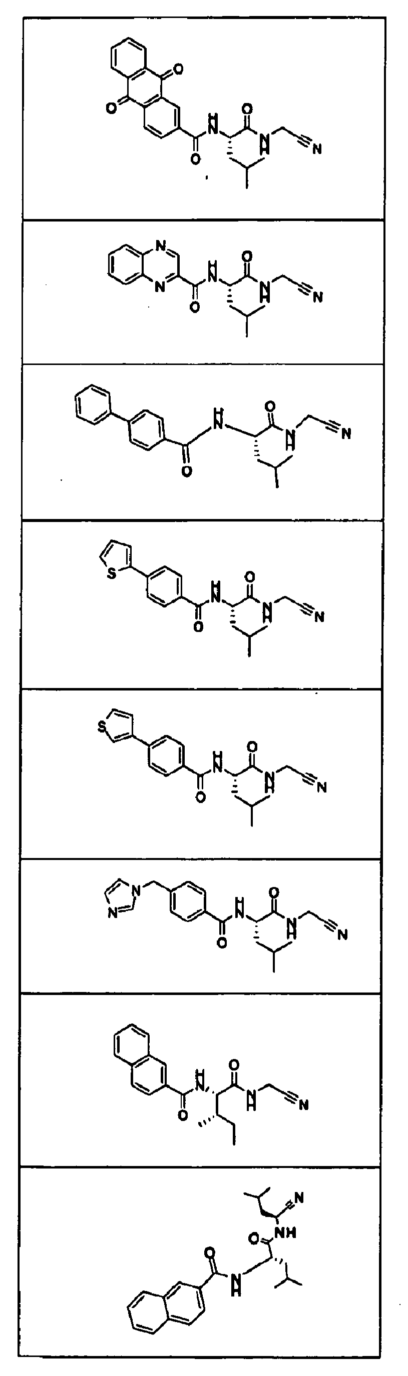

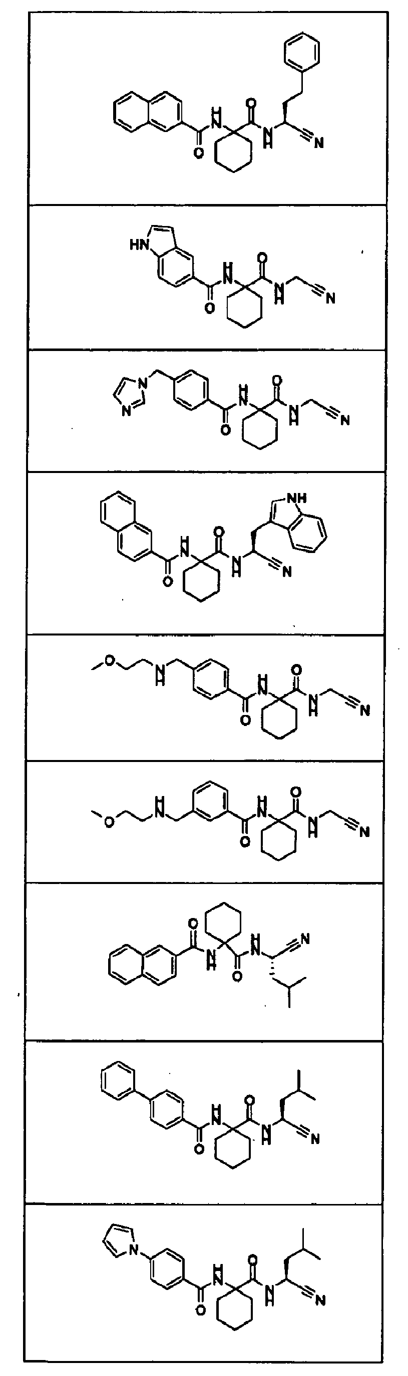

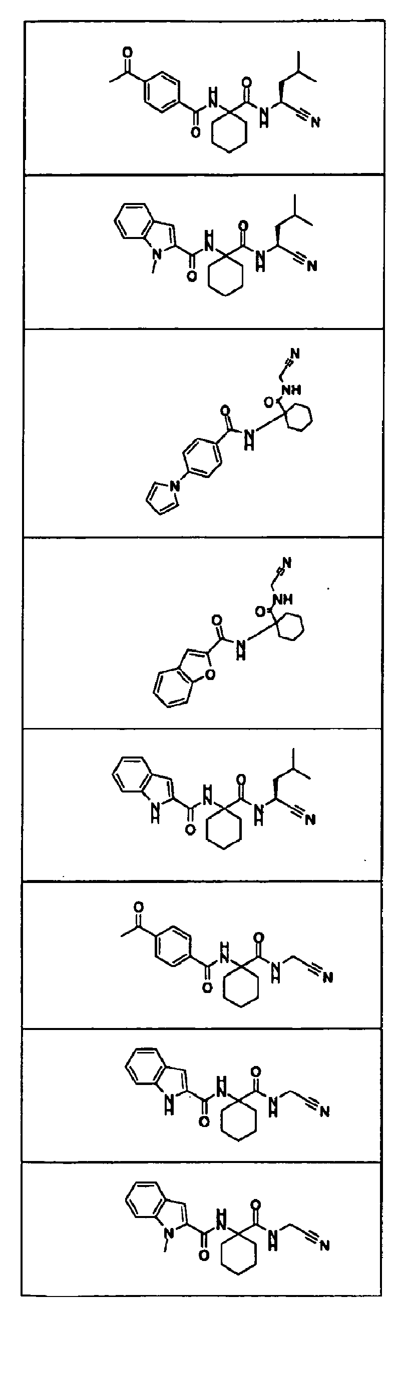

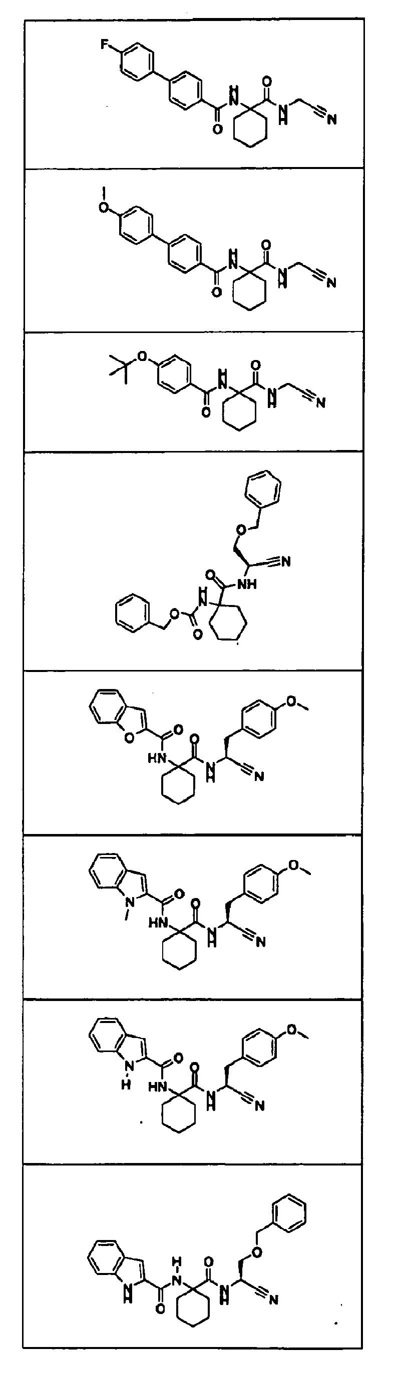

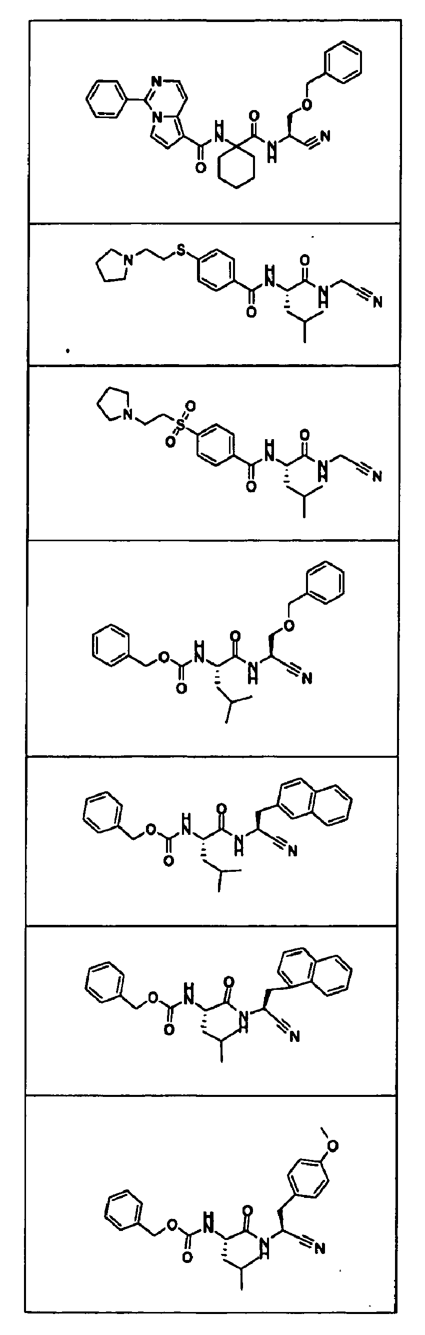

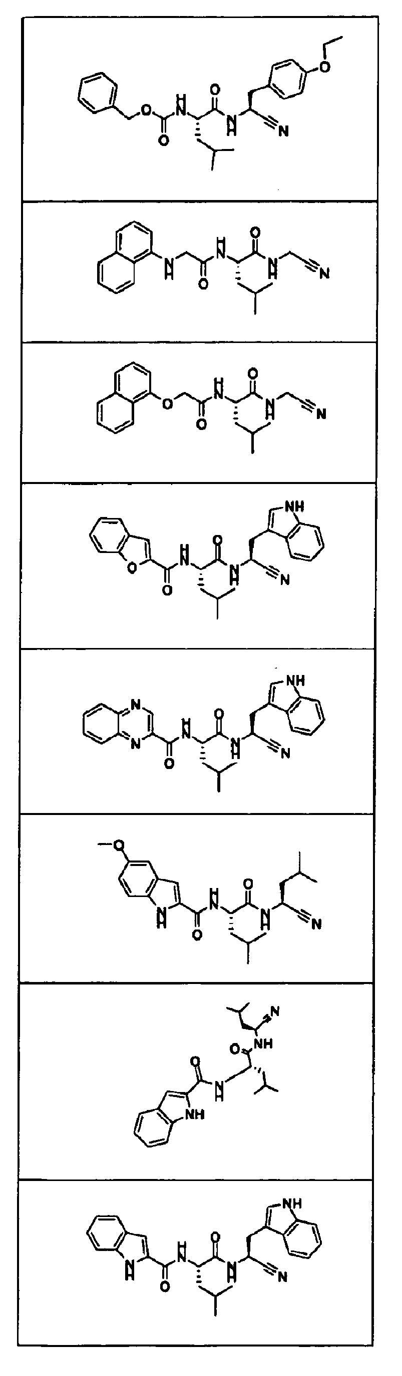

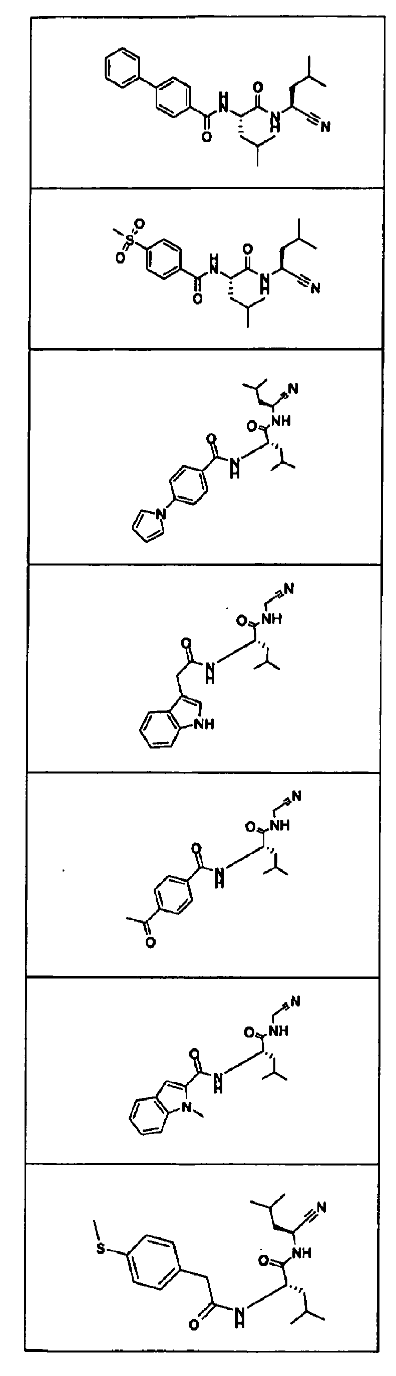

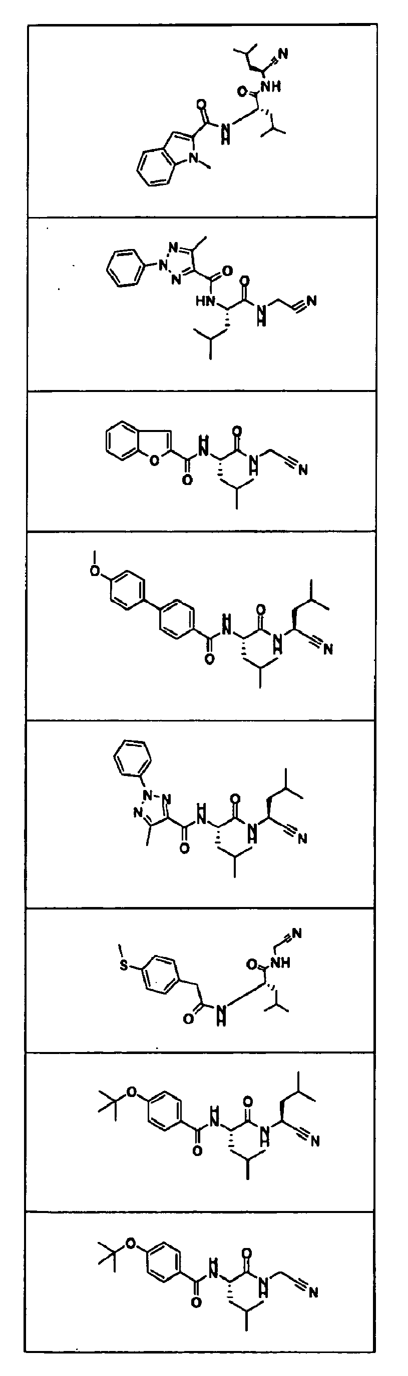

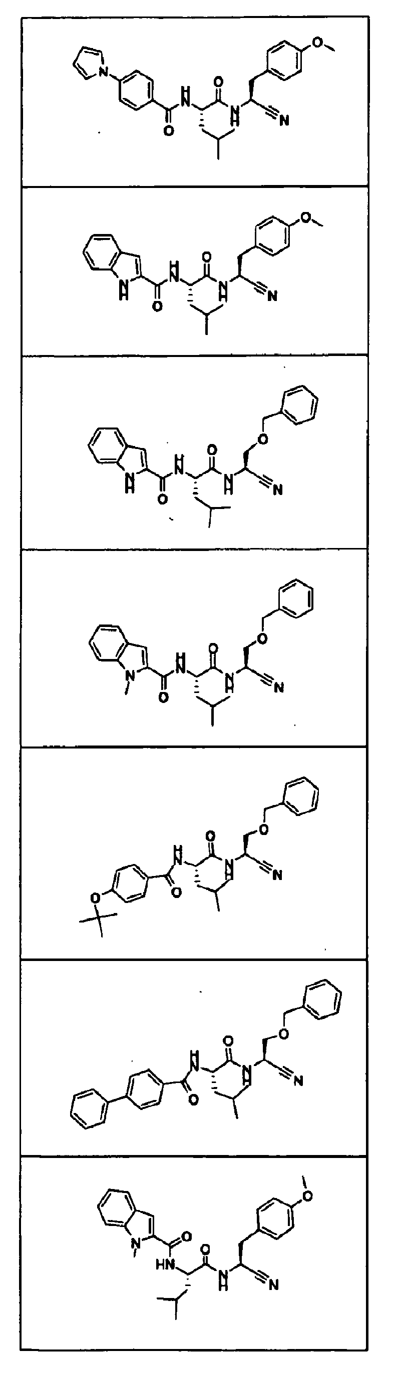

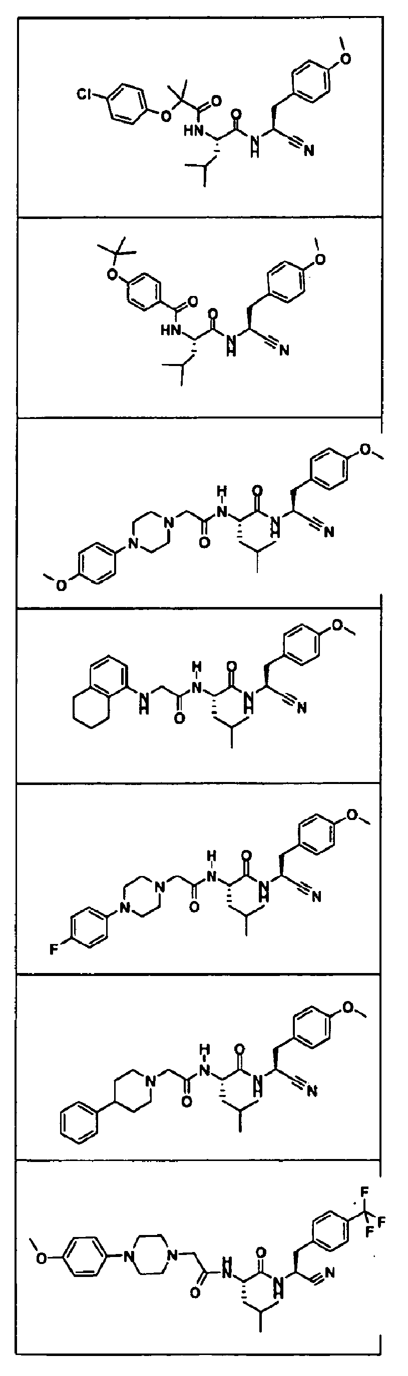

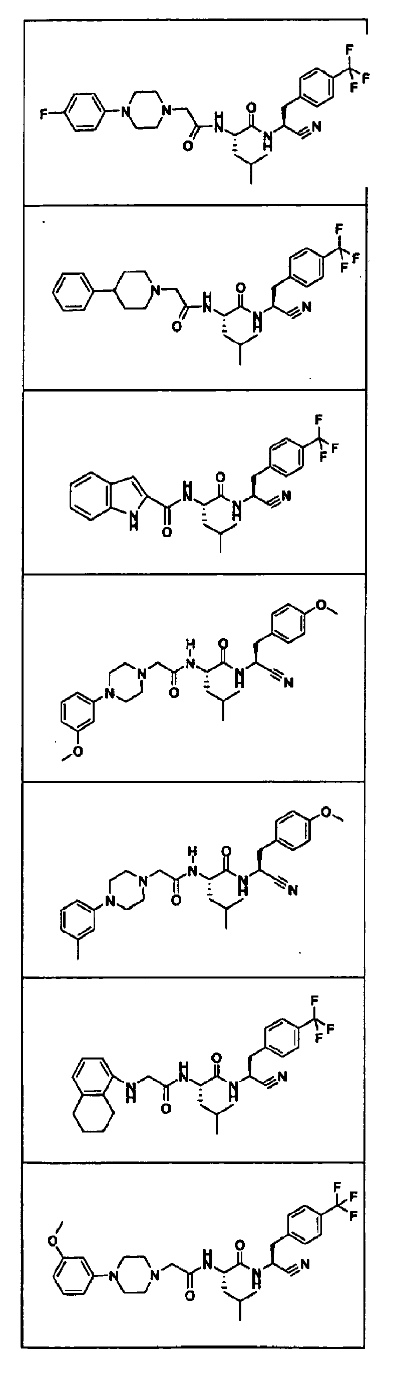

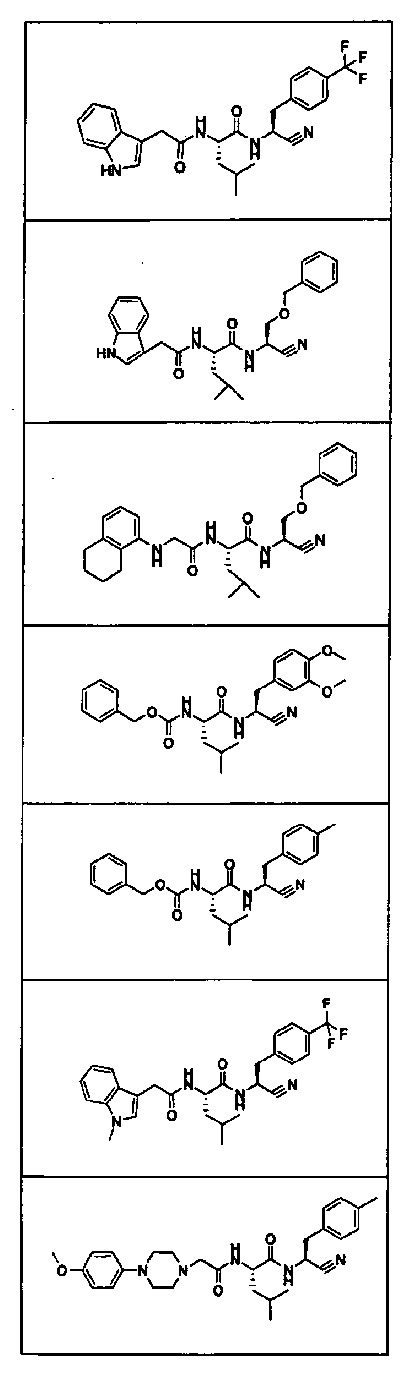

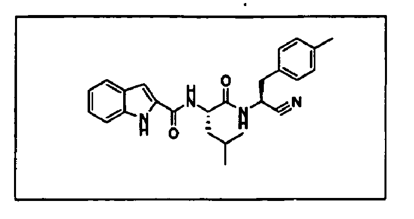

2. Verbindung nach Anspruch 1 zur Verwendung als Medikament.

3. Verwendung einer Verbindung nach Anspruch 1 zur Herstellung eines Medikaments zur Verhinderung oder Behandlung von Osteoporose.

4. Pharmazeutische Zusammensetzung, die eine Verbindung nach Anspruch 1 umfasst.

## Revendications

1. Composé choisi parmi les suivants:

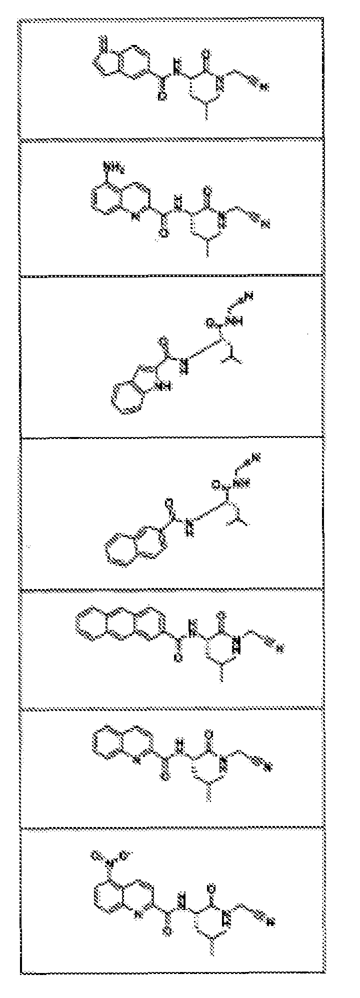

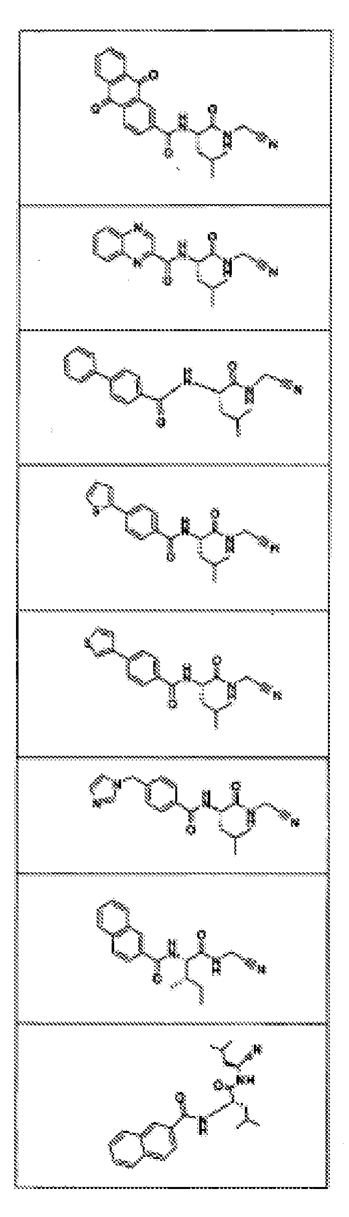

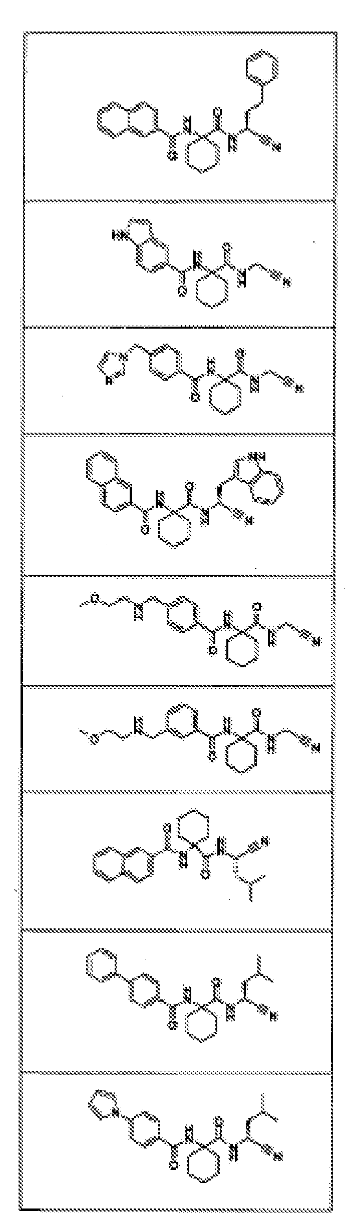

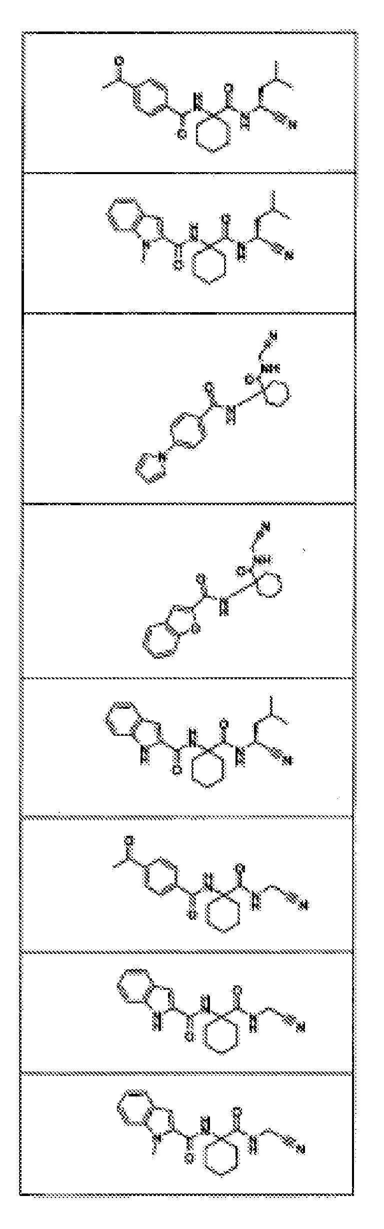

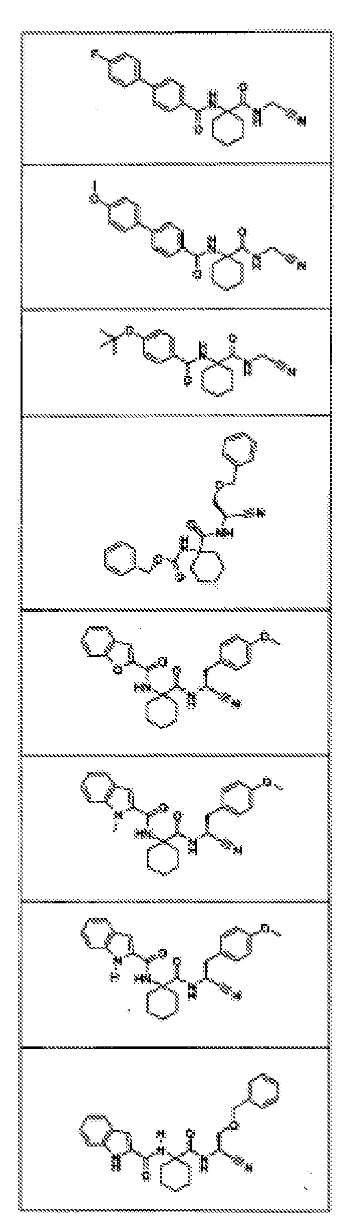

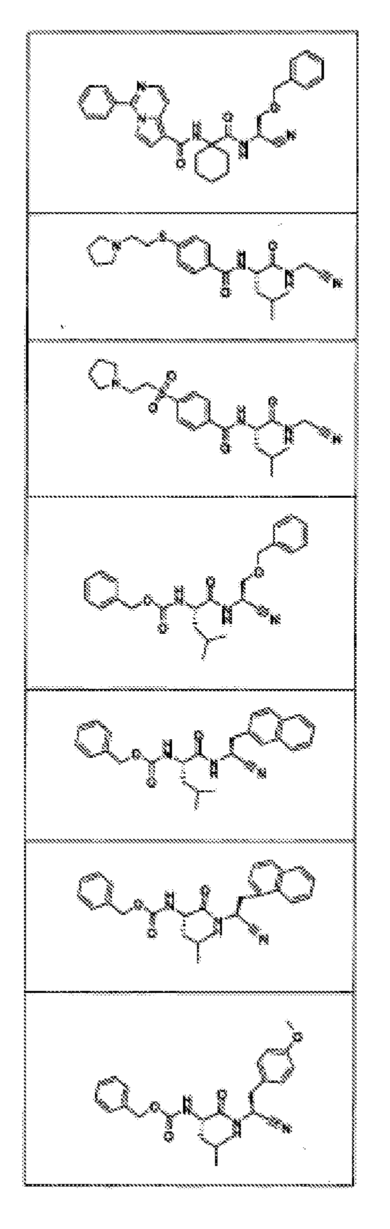

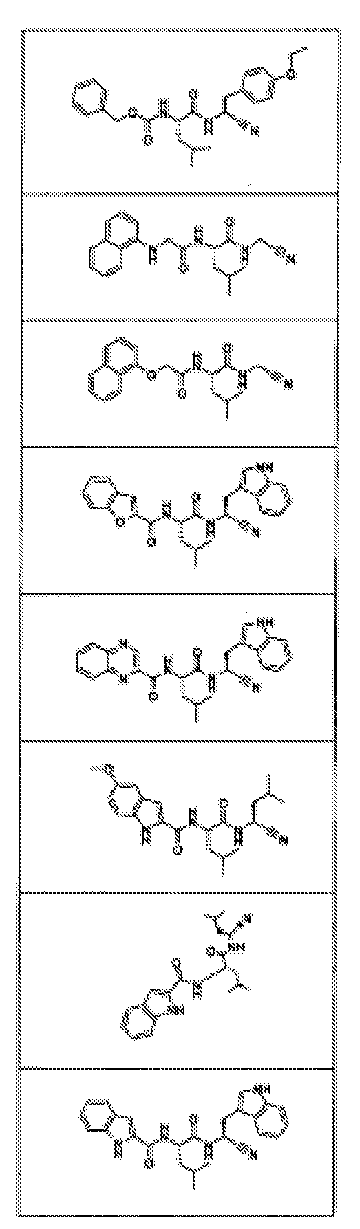

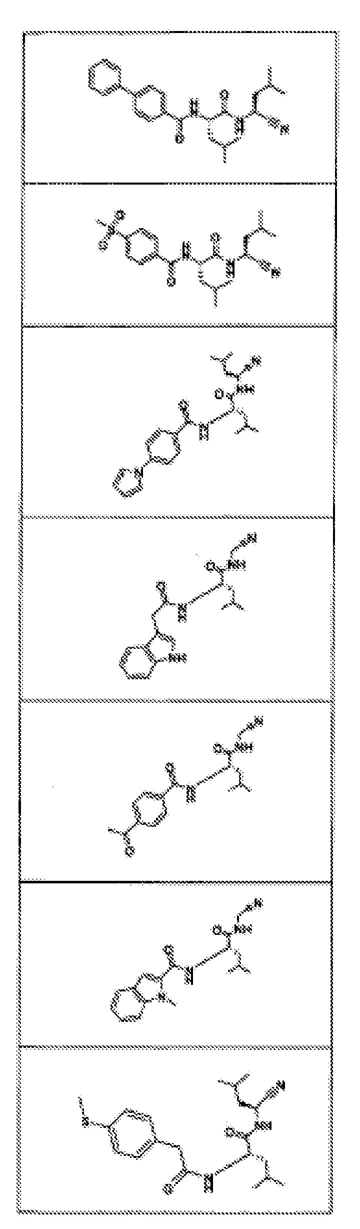

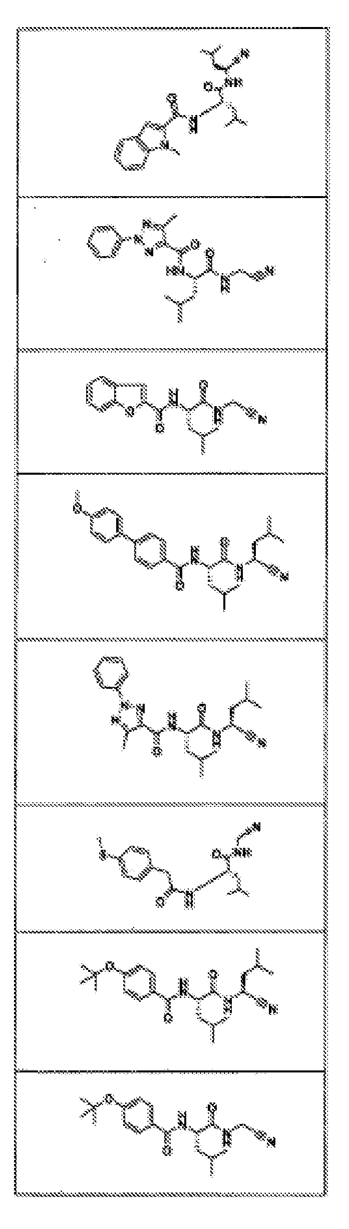

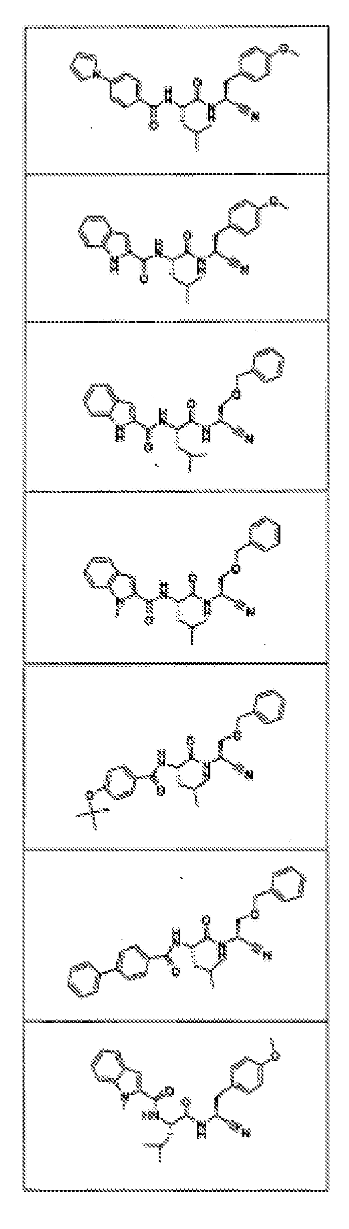

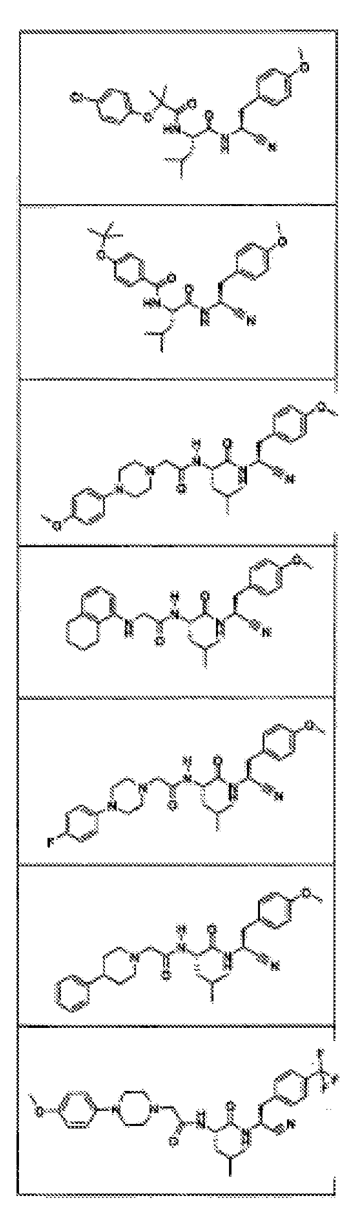

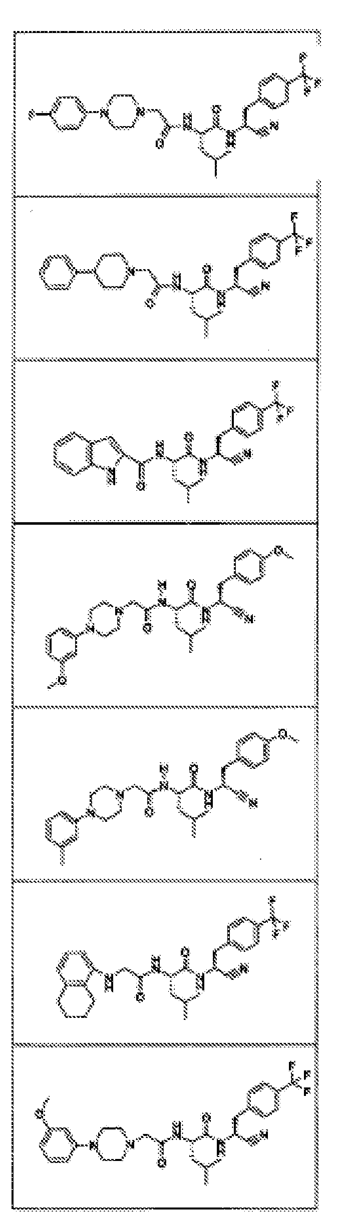

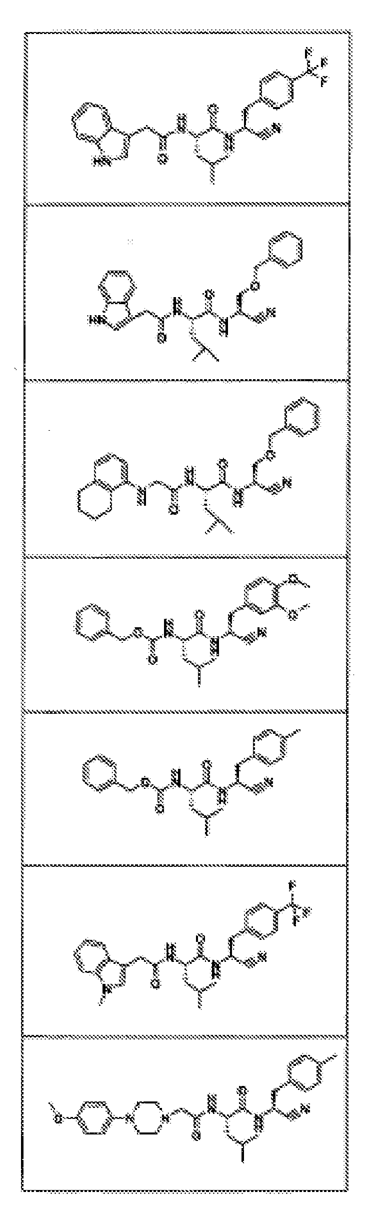

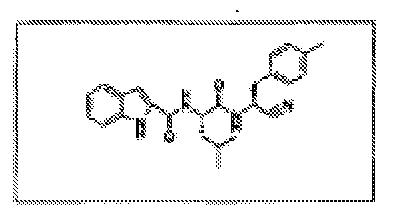

2. Composé selon la revendication 1 à utiliser comme médicament.

3. Utilisation d'un composé selon la revendication 1 pour la préparation d'un médicament destiné à la prévention ou au traitement de l'ostéoporose.

4. Composition pharmaceutique comprenant un composé selon la revendication 1.
